(19)

Europäisches
Patentamt
European
Patent Office
Office européen
des brevets

(11) **EP 3 187 967 B1**

(12) **EUROPEAN PATENT SPECIFICATION**

(45) Date of publication and mention
of the grant of the patent:
**13.02.2019 Bulletin 2019/07**

(21) Application number: **14900520.9**

(22) Date of filing: **15.12.2014**

(51) Int Cl.:
*G06F 3/01* (2006.01)          *G06K 9/00* (2006.01)
*A61B 5/0488* (2006.01)        *G06N 99/00* (2019.01)
*A61B 5/00* (2006.01)          *A61B 5/04* (2006.01)

(86) International application number:
**PCT/CN2014/093793**

(87) International publication number:
**WO 2016/029597 (03.03.2016 Gazette 2016/09)**

(54) **TERMINAL CONTROL METHOD AND SYSTEM**

ENDGERÄTESTEUERUNGSVERFAHREN UND -SYSTEM

PROCÉDÉ ET SYSTÈME DE COMMANDE DE TERMINAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.08.2014 CN 201410422993**

(43) Date of publication of application:
**05.07.2017 Bulletin 2017/27**

(73) Proprietor: SHENZHEN TCL NEW TECHNOLOGY
CO., LTD
**Nanshan District
Shenzhen,
Guangdong 518052 (CN)**

(72) Inventors:
• **LI, Bo
Shenzhen
Guangdong 518052 (CN)**
• **HU, Tian
Shenzhen
Guangdong 518052 (CN)**
• **KANG, Junping
Shenzhen
Guangdong 518052 (CN)**

(74) Representative: **Kuhnen & Wacker
Patent- und Rechtsanwaltsbüro PartG mbB
Prinz-Ludwig-Straße 40A
85354 Freising (DE)**

(56) References cited:
**EP-A1- 1 408 443          CN-A- 1 818 841
CN-A- 101 930 285          US-A1- 2009 327 171**

• **M. B. I. REAZ ET AL: "Techniques of EMG signal
analysis: detection, processing, classification
and applications", BIOLOGICAL PROCEDURES
ONLINE, vol. 13, no. 2, 23 March 2006
(2006-03-23) , pages 12-35, XP055220153, DOI:
10.1251/bpo115**
• **ZHANG XU ET AL: "An Adaptive Feature
Extractor for Gesture SEMG Recognition", 4
January 2008 (2008-01-04), ECCV 2016
CONFERENCE; [LECTURE NOTES IN
COMPUTER SCIENCE; LECT.NOTES
COMPUTER], SPRINGER INTERNATIONAL
PUBLISHING, CHAM, PAGE(S) 83 - 90,
XP047402725, ISSN: 0302-9743 ISBN:
978-3-642-33485-6 * page 83 - page 88 ***
• **BAOFENG SUN ET AL: "The Pattern Recognition
of Surface EMG Based on Wavelet Transform and
BP Neural Network", BIOINFORMATICS AND
BIOMEDICAL ENGINEERING, (ICBBE) 2011 5TH
INTERNATIONAL CONFERENCE ON, IEEE, 10
May 2011 (2011-05-10), pages 1-4, XP031878745,
DOI: 10.1109/ICBBE.2011.5780259 ISBN:
978-1-4244-5088-6**

EP 3 187 967 B1

## Description

TECHNICAL FIELD

The subject matter relates to intelligent control, and particularly, to a terminal control method and a terminal control system.

DESCRIPTION OF RELATED ART

With the development of technology, terminals such as televisions and air conditioners are becoming more and more intelligent. A terminal is usually controlled by a remote controller. A typical remote controller (no matter a universal remote controller or a remote controller adapted to the terminal) controls the terminals via multiple buttons when being operated. The multiple buttons may increase a size of the remote controller and make it inconvenient to be taken with.

EP 1 408 443 A1 describes a method that involves time dividing a time-evolving signal into subframes and determining a parameter value yielded by the signal over a part of the sub-frame. The parameter value is expressed as a component of a vector along a dimension specifically allocated to that sub-frame. The resultant vector forms a signature of a gesture. The value determination and expressing the value as a vector component are performed cyclically.

US 2009/327171 A1 describes: a machine learning model is trained by instructing a user to perform proscribed gestures, sampling signals from EMG sensors arranged arbitrarily on the user's forearm with respect to locations of muscles in the forearm, extracting feature samples from the sampled signals, labeling the feature samples according to the corresponding gestures instructed to be performed, and training the machine learning model with the labeled feature samples. Subsequently, gestures may be recognized using the trained machine learning model by sampling signals from the EMG sensors, extracting from the signals unlabeled feature samples of a same type as those extracted during the training, passing the unlabeled feature samples to the machine learning model, and outputting from the machine learning model indicia of a gesture classified by the machine learning model.

In "Techniques of EMG signal analysis: detection, processing, classification and applications", M. B. I. REAZ ET AL describes that Electromyography (EMG) signals can be used for clinical/biomedical applications, Evolvable Hardware Chip (EHW) development, and modem human computer interaction. EMG signals acquired from muscles require advanced methods for detection, decomposition, processing, and classification. This paper illustrates the various methodologies and algorithms for EMG signal analysis to provide efficient and effective ways of understanding the signal and its nature. They further point up some of the hardware implementations using EMG focusing on applications related to prosthetic hand control, grasp recognition, and human computer interaction. A comparison study is also given to show performance of various EMG signal analysis methods.

In "An Adaptive Feature Extractor for Gesture SEMG Recognition", ZHANG XU ET AL proposes an adaptive feature extraction method for pattern recognition of hand gesture action sEMG to enhance the reusability of myoelectric control. The feature extractor is based on wavelet packet transform and Local Discriminant Basis (LDB) algorithms to select several optimized decomposition subspaces of origin SEMG waveforms caused by hand gesture motions. Then the square roots of mean energy of signal in those subspaces are calculated to form the feature vector. In data acquisition experiments, five healthy subjects implement six kinds of hand motions every day for a week. The recognition results of hand gesture on the basis of the measured SEMG signals from different use sessions demonstrate that the feature extractor is effective.

In "The Pattern Recognition of Surface EMG Based on Wavelet Transform and BP Neural Network" paper, BAOFENG SUN ET AL use both wavelet transform and BP neural network to identify SEMG from human upper arm. In the experiments, they decompose a single action into different parts to realize the multi-level identification of a single action. They use two electrodes to extract SEMG signal from the upper arm biceps, triceps firstly, then analyze this signal using wavelet transform and extract eight values forming the feature vector, finally put this feature vectors into BP neural network to complete pattern recognition. The results of the experiments using the method introduced in this paper show that the average recognition rate of arm internal rotating, external rotating, arm stretching, 1/3 bending, 1/2 bending and full bending is over 90%.

BRIEF SUMMARY OF THE DISCLOSURE

A main object of the present disclosure is to overcome the above problem.

The present invention is defined in and by the appended claims.

Also described herein is a terminal control method which includes following steps.

When an electromyographic signal is detected by an electromyographic signal detecting device, the terminal obtains a characteristic value of the electromyographic signal, the characteristic value is an average value or a variance of wavelet coefficients obtained by performing wavelet decomposition of the electromyographic signal;

The terminal searches the mapping relationship between preset characteristic values and control codes to determine whether there is a preset characteristic value which can match the obtained characteristic value, if yes, the terminal determines a control code corresponding to the matching preset characteristic value;

The terminal responds to a control signal corresponding to the determined control code.

Preferably, the step of "when an electromyographic signal is detected by an electromyographic signal detecting device, the terminal obtaining a characteristic value of the electromyographic signal" further includes following steps.

When the electromyographic signal is detected by the electromyographic signal detecting device, the terminal samples the electromyographic signal to obtain a number of sample points and obtains an energy value of each sample point;

When each of the energy values of a number of successive sample points is greater than a first energy threshold, and when the number of the successive sample points is greater than a first number threshold, the terminal sets a start point of a first segment of the electromyographic signal including the successive sample points as a start point of an effective electromyographic signal;

When each of the energy values of a number of successive sample points is less than a second energy threshold, and when the number of the successive sample points is greater than a second number threshold, the terminal sets an end point of a second segment of the electromyographic signal including the successive sample points as an end point of the effective electromyographic signal;

The terminal extracts the characteristic value of the effective electromyographic signal between the start point and the end point.

Preferably, the step of "when each of the energy values of a number of successive sample points is greater than a first energy threshold, and when the number of the successive sample points is greater than a first number threshold, the terminal setting a start point of a first segment of the electromyographic signal including the successive sample points as a start point of an effective electromyographic signal" further includes following steps.

When each of the energy values of a number of successive sample points is greater than the first energy threshold, and when the number of the successive sample points is greater than the first number threshold, the terminal obtains the energy value of the first segment of the electromyographic signal including the successive sample points;

When the energy value of the first segment of the electromyographic signal is greater than the first energy threshold, the terminal sets the start point of the first segment of the electromyographic signal as the start point of the effective electromyographic signal.

Preferably, the step of "when each of the energy values of a number of successive sample points is less than a second energy threshold, and when the number of the successive sample points is greater than a second number threshold, the terminal setting an end point of a second segment of the electromyographic signal including the successive sample points as an end point of an effective electromyographic signal" further includes following steps.

When each of the energy values of a number of successive sample points is less than the second energy threshold, and when the number of the successive sample points is greater than the second number threshold, the terminal obtains the energy value of the second segment of the electromyographic signal including the successive sample points;

When the energy value of the second segment of the electromyographic signal is less than the second energy threshold, the terminal sets the end point of the second segment of the electromyographic signal as the end point of the effective electromyographic signal.

Preferably, learning the mapping relationship between the preset characteristic values and the control codes further includes following steps.

When a gesture learning command is detected, the terminal displays a selecting interface including a number of standard gesture pictures for a user to select a standard gesture picture, and triggers a selecting command after the standard gesture picture is selected;

When the selecting command is received, the terminal is locked into displaying the corresponding standard gesture picture according to the selecting command;

When the electromyographic signal is detected by the electromyographic signal detecting device, the terminal obtains the characteristic value of the electromyographic signal;

The terminal determines a control code corresponding to the standard gesture picture currently displayed, and updates the characteristic value corresponding to the determined control code to the obtained characteristic value.

Preferably, the terminal control method further includes following steps for learning the mapping relationship between the preset characteristic values and the control codes.

When a gesture learning command is detected, the terminal detects the electromyographic signal from the electromyographic signal detecting device;

When the electromyographic signal is detected by the electromyographic signal detecting device, the terminal obtains the characteristic value of the electromyographic signal and receives a control code from a control terminal;

When the control code is received from the control terminal, the terminal associates the obtained characteristic value with the received control code, and stores the obtained characteristic value and the associated control code to the mapping relationship between the characteristic values and the control codes.

Preferably, the terminal control method further includes following steps before the step of "when a gesture learning command is detected, the terminal detecting an electromyographic signal from an electromyographic signal detecting device".

When the terminal detects a same electromyographic signal for a number of times within a preset time interval and the number of times is greater than a preset threshold, the terminal triggers the gesture learning command.

Preferably, the method further includes following steps for learning the mapping relationship between the preset characteristic values and the control codes.

When the gesture updating command is detected, the terminal successively displays the standard gesture pictures;

After each standard gesture picture is displayed, the terminal detects the electromyographic signal;

When the electromyographic signal is detected, the terminal determines a control code corresponding to the standard gesture picture currently displayed;

The terminal updates the characteristic value corresponding to the determined control code to the obtained characteristic value, and displays a next standard gesture picture and continues to learn the mapping relationship.

Preferably, the step of "the terminal searching the mapping relationship between preset characteristic values and control codes to determine whether there is a preset characteristic value which can match the obtained characteristic value, if yes, the terminal determining a control code corresponding to the matching preset characteristic value" further includes following steps.

The terminal determines an identifier of the electromyographic signal detecting device according to the detected electromyographic signal;

The terminal obtains the mapping relationship between a preset characteristic value corresponding to the identifier and the control code, according to the mapping relationship between the preset characteristic values and the control codes, and according to an association between the identifiers;

When a characteristic value matches the preset characteristic value in the mapping relationship between the preset characteristic values and the control codes, the terminal determines a control code corresponding to the matching preset characteristic value.

Further described herein is a terminal control system which includes the following modules.

An obtaining module is configured to obtain a characteristic value of the electromyographic signal when the electromyographic signal is detected by an electromyographic signal detecting device, the characteristic value is an average value or a variance of wavelet coefficients obtained by performing wavelet decomposition of the electromyographic signal;

A determining module is configured to search the mapping relationship between preset characteristic values and control codes to determine whether there is a preset characteristic value which can match the obtained characteristic value, if yes, the determining module determines a control code corresponding to the matching preset characteristic value;

A responding module is configured to respond to a control signal corresponding to the determined control code.

Preferably, the obtaining module includes following sub-modules.

A sampling sub-module is configured to sample the detected electromyographic signal to obtain a number of sample points when the electromyographic signal is detected by the electromyographic signal detecting device;

An energy obtaining sub-module is configured to obtain an energy value of each sample point;

A start point obtaining sub-module is configured to set a start point of a first segment of the electromyographic signal including the successive sample points as a start point of an effective electromyographic signal, when each of the energy values of a number of successive sample points is greater than a first energy threshold, and when the number of the successive sample points is greater than a first number threshold;

An end point obtaining sub-module is configured to set an end point of a second segment of the electromyographic signal including a plurality of successive sample points as an end point of an effective electromyographic signal, when each of the energy values of a number of successive sample points is less than a second energy threshold, and when the number of the successive sample points is greater than a second number threshold;

An extracting sub-module is configured to extract the characteristic value of the effective electromyographic signal between the start point and the end point.

Preferably, the start point obtaining sub-module further includes following units.

A first energy value obtaining unit is configured to obtain the energy value of the first segment of the electromyographic signal including the successive sample points, when each of the energy values of successive sample points is greater than the first energy threshold, and when the number of the successive sample points is greater than the first number threshold;

A start point obtaining unit is configured to set the start point of the first segment of the electromyographic signal as the start point of the effective electromyographic signal, when the energy value of the first segment of the electromyographic signal is greater than the first energy threshold.

Preferably, the end point obtaining sub-module further includes following units.

A second energy value obtaining unit is configured to obtain the energy value of the second segment of the electromyographic signal including the successive sample points, when each of the energy values of the successive sample points is less than the second energy threshold, and when the number of the successive sample points is greater than the second number threshold;

An end point obtaining unit is configured to set the end point of the second segment of the electromyographic signal as the end point of the effective electromyographic signal, when the energy value of the second segment of the electromyographic signal is less than the second energy threshold.

Preferably, the terminal control system includes following modules.

A display module is configured to display a selecting interface including a number of standard gesture pictures for the user to select a standard gesture picture and, and trigger a selecting command after the standard gesture picture is selected, when a gesture learning command is detected;

A locking module is configured to lock the terminal into displaying the corresponding standard gesture picture according to the selecting command, when the selecting command is received;

The obtaining module is further configured to obtain the characteristic value of the electromyographic signal, when the electromyographic signal is detected by the electromyographic signal detecting device;

An updating module is configured to determine a control code corresponding to the standard gesture picture currently displayed, and update the characteristic value corresponding to the determined control code to the obtained characteristic value.

Preferably, the terminal control system further includes following module.

A detecting module is configured to detect the electromyographic signal from the electromyographic signal detecting device, when the gesture learning command is detected;

The obtaining module is further configured to obtain the characteristic value of the electromyographic signal and receive the control code from the control terminal, when the electromyographic signal is detected by the electromyographic signal detecting device;

A storage module is configured to associate the obtained characteristic value with the received control code when the control code is received from the control terminal, and store the obtained characteristic value and the associated control code to the mapping relationship between the characteristic values and the control codes.

Preferably, the terminal control system further includes following modules.

A display module is configured to successively display the standard gesture pictures when the gesture learning command is detected;

A detecting module is configured to detect the electromyographic signal after each standard gesture picture is displayed;

A determining module is configured to determine the control code corresponding to the standard gesture picture currently displayed when the electromyographic signal is detected;

An updating module is configured to update the characteristic value corresponding to the determined control code to the obtained characteristic value.

The display module is further configured to display a next standard gesture picture and continue to learn the mapping relationship.

Preferably, the determining module further includes following units.

An obtaining unit is configured to determine the identifier of the electromyographic signal detecting device according to the detected electromyographic signal, obtain a mapping relationship between a preset characteristic value corresponding to the identifier and the control code, according to the mapping relationship between the preset characteristic values and the control codes, and according to an association between the identifiers;

A determining unit is configured to determine the control code corresponding to the matching preset characteristic value, when a characteristic value matches the preset characteristic value in the mapping relationship between the preset characteristic values and the control codes.

In the terminal control method and system of the present disclosure, the terminal can obtain the characteristic value of the detected electromyographic signal, determines the control code corresponding to the obtained characteristic value, and respond to the determined control code, thereby allowing the terminal to be controlled by a gesture. The electromyographic signal detecting device has no need to employ multiple unnecessary mechanical buttons, but can send the electromyographic signal based on the gesture of the use, thereby decreasing the size thereof and making it portable and flexible to be controlled.

BRIEF DESCRIPTION OF THE DRAWINGS

Fig. 1 is a flowchart of an embodiment of a terminal control method.
FIG. 2 is a sub-flowchart of step S10 of the terminal control method of FIG. 1.
FIG. 3 is a flowchart of a first embodiment of a method for learning a mapping relationship between preset characteristic values and control codes in the terminal control method of FIG. 1.
FIG. 4 is a flowchart of a second embodiment of a method for learning the mapping relationship between the preset characteristic values and the control codes in the terminal control method of FIG. 1.
FIG. 5 is a sub-flowchart of step S12 of the terminal control method of FIG. 2.
FIG. 6 is a sub-flowchart of step S13 of the terminal control method of FIG. 2.
FIG. 7 is a block diagram of an embodiment of a terminal control system.
FIG. 8 is a block diagram of an obtaining module included in the terminal control system of FIG. 7.
FIG. 9 is a block diagram of a first embodiment of the terminal control system of FIG. 7, which can learn the mapping relationship between the preset characteristic values and the control codes.

FIG. 10 is a block diagram of a second embodiment of the terminal control system of FIG. 7, which can learn the mapping relationship between the preset characteristic values and the control codes.

FIG. 11 is a block diagram of a start point obtaining sub-module included in the terminal control system of FIG. 8.

FIG. 12 is a block diagram of an end point obtaining sub-module included in the terminal control system of FIG. 8.

The technical solution of the present invention is hereinafter described in detail with reference to the accompanying drawings.

DETAILED DESCRIPTION

It should be understood that the embodiments described herein are only intended to illustrate, but not to limit the present disclosure.

The present disclosure provides a terminal control method.

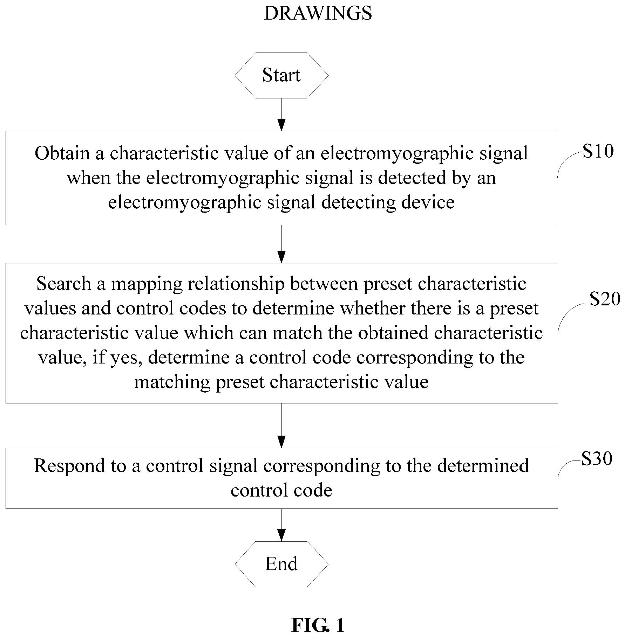

Referring to FIG. 1, FIG. 1 is a flowchart of an embodiment of the terminal control method.

The terminal control method includes following steps.

At step S10, when an electromyographic signal is detected by an electromyographic signal detecting device, the terminal obtains a characteristic value of the electromyographic signal. The characteristic value is an average value or a variance of wavelet coefficients obtained by performing wavelet decomposition of the electromyographic signal.

In this embodiment, the electromyographic signal detecting device can be employed inside a wristband or a wristwatch to detect a gesture of a user. When the electromyographic signal detecting device detects the electromyographic signal, the electromyographic signal detecting device sends the detected electromyographic signal to the terminal.

To obtain the characteristic value, the electromyographic signal is performed by wavelet decomposition, and an average value or a variance of wavelet coefficients is calculated. That is, the characteristic value is an average value or a variance of wavelet coefficients obtained by performing wavelet decomposition of the electromyographic signal. The calculation of the wavelet coefficient and the variance thereof is well-known.

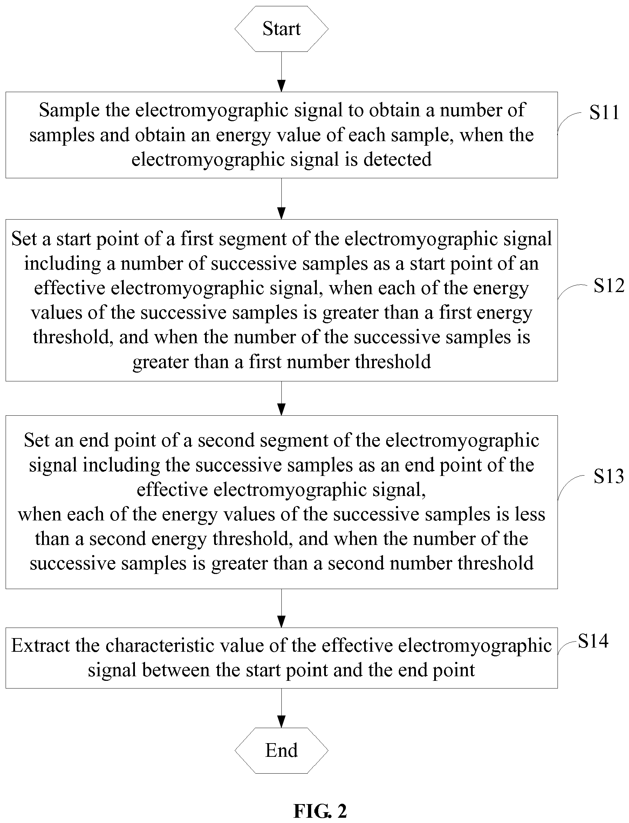

FIG. 2 illustrates that the above step S10 of the terminal obtaining the detected electromyographic signal further includes following steps.

At step S11, when the electromyographic signal is detected by the electromyographic signal detecting device, the terminal samples the electromyographic signal to obtain a number of sample points and obtains an energy value of each sample point.

To maintain the accuracy of the obtained electromyographic signal, when sampling the electromyographic signal, the electromyographic signal can first be filtered and denoised. For example, the frequency of a common electromyographic signal ranges from 20HZ to 500HZ, the detected electromyographic signal can be filtered via a band-pass filter which is used to filter signals with a frequency less than 20HZ and greater than 500HZ. The denoise of the electromyographic signal is well-known.

In this embodiment, the electromyographic signal can be sampled by multiple ways. Example a: the electromyographic signal is sampled at each preset time interval until a preset number of sample points are obtained, and the preset time interval is determined based on a lasting duration of the electromyographic signal; Example b: the electromyographic signal is sampled at each preset time interval until a preset number of sample points are obtained, and the obtained sample points are further sampled to obtain the final sample points, for example, the electromyographic signal is sampled to obtain one thousand sample points, and the one thousand sample points are further sampled to obtain the final five hundred sample points. What described above are only preferred embodiments of the present disclosure. Any equivalent modifications for sampling the electromyographic signal according to need shall also fall within the scope of the present disclosure.

In this embodiment, since the electromyographic signal is presented as a waveform, the energy value of each sample point equals to a square value of the amplitude of a point included in the waveform corresponding to the sample point.

At step S12, when each of the energy values of a number of successive sample points is greater than a first energy threshold, and when the number of the successive sample points is greater than a first number threshold, the terminal sets a start point of a first segment of the electromyographic signal including the successive sample points as a start point of an effective electromyographic signal.

In this embodiment, when each of the energy values of the successive sample points is greater than the first energy threshold, the electromyographic signal may start from the successive sample points. Then, the start point of the first segment of the electromyographic signal including the successive sample points is set as the start point of the electromyographic signal. The first energy threshold and the first number threshold are preset, and the first energy threshold can be determined by an average value of the energy values of the start points of the electromyographic signals corresponding to different gestures.

At step S13, when each of the energy values of a number of successive sample points is less than a second energy threshold, and when the number of the successive sample points is greater than a second number threshold, the terminal sets an end point of a second segment of the electromyographic signal including the successive sample points as an end point of the effective electromyographic signal.

In this embodiment, when each of the energy values of the successive sample points is less than the second energy threshold, the electromyographic signal may disappear gradually. Then, the end point of the second segment of the electromyographic signal including the successive sample points is set as the end point of the effective electromyographic signal. The second energy threshold and the second number threshold are preset, and the second energy threshold can be determined by an average value of the energy values of the end points of the electromyographic signals corresponding to different gestures. The second energy value can equal to the first energy value, and can also be different from the first energy value.

At step S14, the terminal extracts the characteristic value of the effective electromyographic signal between the start point and the end point.

To extract the characteristic value, the electromyographic signal is performed by wavelet decomposition, and an average value or a variance of wavelet coefficients is calculated to obtain the characteristic value. The calculation of the wavelet coefficient and the variance thereof is well-known.

At step S20, the terminal searches the mapping relationship between preset characteristic values and control codes to determine whether there is a preset characteristic value which can match the obtained characteristic value, if yes, the terminal determines a control code corresponding to the matching preset characteristic value.

In this embodiment, since the wristband or the wristwatch employing the electromyographic signal detecting device may move during the gesture is performed by the user, the detected electromyographic signal may fluctuate. When there is a preset characteristic value which can match the obtained characteristic value, a difference between the preset characteristic value and the obtained characteristic value is less than a preset threshold.

Furthermore, the electromyographic signals corresponding to a same gesture performed by different users may be different, that is, the preset characteristic values and the control codes of different users may be different. To improve the flexibility of the terminal control, the above step S20 further includes following steps. The terminal determines the identifier of the electromyographic signal detecting device according to the obtained electromyographic signal, obtains a mapping relationship between a preset characteristic value corresponding to the identifier and the control code, according to the mapping relationship between the preset characteristic values and the control codes, and according to an association between the identifiers. When a characteristic value matches the preset characteristic value in the mapping relationship between the preset characteristic values and the control codes, the terminal determines a control code corresponding to the matching preset characteristic value.

At step S30, the terminal responds to a control signal corresponding to the determined control code.

In the terminal control method and system of the present disclosure, the terminal can obtain the characteristic value of the detected electromyographic signal, determines the control code corresponding to the obtained characteristic value, and respond to the determined control code, thereby allowing the terminal to be controlled by a gesture. The electromyographic signal detecting device has no need to employ multiple unnecessary mechanical buttons, but can send the electromyographic signal based on the gesture of the user, thereby decreasing the size thereof and making it portable and flexible to be controlled.

In the terminal control method of the present disclosure, presetting the mapping relationship between the preset characteristic values and the control codes includes following steps.

a. The terminal collects the electromyographic signal and preprocesses the collected electromyographic signal.

b. The terminal samples the preprocessed electromyographic signal to obtain a number of sample points, and obtains the energy value of each sample point. When each of the energy values of the successive sample points is greater than the first energy threshold, and when the number of the successive sample points is greater than the first number threshold, the terminal sets the start point of the first segment of the electromyographic signal as the start point of the effective electromyographic signal. When each of the energy values of a number of successive sample points is less than the second energy threshold, and when the number of the successive sample points is greater than the second number threshold, the terminal sets the end point of the second segment of the electromyographic signal including the successive sample points as the end point of the effective electromyographic signal.

c. The terminal extracts the characteristic value of the effective electromyographic signal between the start point and the end point.

d. The terminal associates the obtained characteristic value with the control code, stores the obtained characteristic value and the associated control code, and generates the mapping relationship between the characteristic values and the control codes based on a training pattern classifier. In this embodiment, the generation of the mapping relationship by the training pattern classifier is well-known.

To improve the accuracy and flexibility of the terminal control, the terminal control method further includes learning the mapping relationship between the preset characteristic values and the control codes.

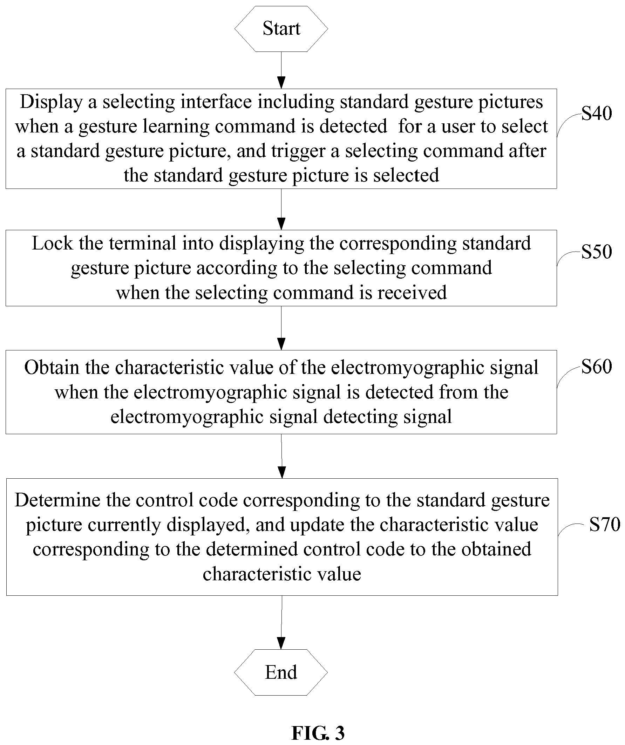

Referring to FIG. 3, FIG. 3 illustrates that a first embodiment of learning the mapping relationship between the preset characteristic values and the control codes includes following steps.

At step S40, when a gesture learning command is detected, the terminal displays a selecting interface including a number of standard gesture pictures for a user to select a standard gesture picture, and triggers a selecting command after the standard gesture picture is selected.

In this embodiment, when terminal fails in detecting the gesture learning command, the user can send the gesture learning command to the terminal by a control terminal (such as a remote controller of a smart phone) or a voice control command. In other embodiments, when the terminal detects a same electromyographic signal for a number of times within a preset time interval and the number of times is greater than a preset threshold, the terminal determines that it fails in detecting the gesture learning command and triggers the gesture learning command.

At step S50, when the selecting command is received, the terminal is locked into displaying the corresponding standard gesture picture according to the selecting command.

At step S60, when the electromyographic signal is detected by the electromyographic signal detecting device, the terminal obtains the characteristic value of the electromyographic signal.

When the terminal obtains the characteristic value of the detected electromyographic signal, the terminal can first filter and denoise the electromyographic signal according to the first embodiment, determines the start point and the end point of the electromyographic signal, treats the electromyographic signal between the start point and the end point as the effective electromyographic signal, and obtains the characteristic value of the effective electromyographic signal.

To improve the accuracy of the learning of the mapping relationship, when the electromyographic signal is detected by the electromyographic signal detecting device, the terminal displays a confirming interface. When a confirming command is detected by the confirming interface, the terminal obtains the characteristic value of the obtained electromyographic signal.

At step S70, the terminal determines the control code corresponding to the standard gesture picture currently displayed, and updates the characteristic value corresponding to the determined control code to the obtained characteristic value.

In this embodiment, the user can send a number of electromyographic signals within a preset time interval. When the terminal detects a number of electromyographic signals within a preset time interval, the terminal can obtain an average value of the characteristic values of the electromyographic signals, and update the control code corresponding to the obtained characteristic value according to the obtained average value.

To improve the accuracy of the terminal control, when the gesture learning command is detected, the terminal can successively display the standard gesture pictures. After each standard gesture picture is displayed, the terminal detects the electromyographic signal. When the electromyographic signal is detected, the terminal determines a control code corresponding to the standard gesture picture currently displayed, updates the characteristic value corresponding to the determined control code to the obtained characteristic value, and displays a next standard gesture picture and continues to learn the mapping relationship.

Since different users have different habits to perform a same standard gesture, the standard gesture performed may thus be imperfect or inaccurate. For example, the user may perform a gesture according to his or her habit by placing the palm upwards about 45 degrees. However, a preset standard gesture needs the user to place the palm upwards about 75 degrees, which may make the gesture performed by the user difficult to be identified. Thus, the user may need to learn the standard gesture according to following steps. The user sends a gesture learning command to the terminal via a control terminal for example. When the terminal receives the gesture learning command, the terminal displays a standard gesture picture interface. The user can select a standard gesture picture of the palm placing upwards 75 degrees via the standard gesture picture interface, and performs a gesture according to his or her habit (for example, the user place the palm upwards about 45 degrees). Then, the electromyographic signal detecting device sends the electromyographic signal to the terminal. The terminal obtains the characteristic value of the electromyographic signal, determines a control code corresponding to the standard gesture picture of the palm placing upwards 75 degrees, and updates the characteristic value corresponding to the determined control code to the obtained characteristic value corresponding to the standard gesture picture of the palm placing upwards about 45 degrees.

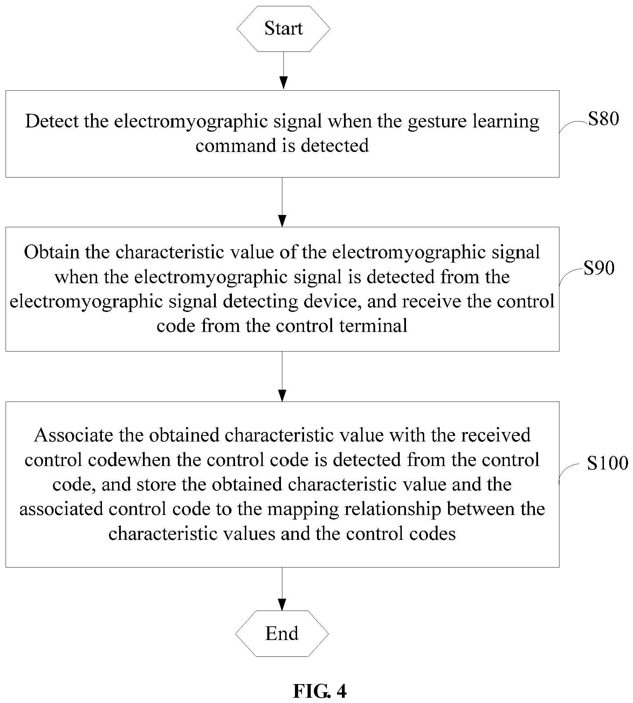

FIG. 4 illustrates a second embodiment of learning the mapping relationship between the preset characteristic values and the control codes including following steps.

At step S80, when the gesture learning command is detected, the terminal detects the electromyographic signal from the electromyographic signal detecting device.

In this embodiment, when terminal fails in detecting the gesture learning command, the user can trigger the gesture learning command by a control terminal (such as a remote controller of a smart phone) or a voice control command. In other embodiments, when the terminal detects a same electromyographic signal for a number of times within a preset time interval and the number of times is greater than a preset threshold, the terminal determines that it fails in detecting the gesture learning command and triggers the gesture learning command.

At step S90, when the electromyographic signal is detected by the electromyographic signal detecting device, the terminal obtains the characteristic value of the electromyographic signal, and receives the control code from the control terminal.

In this embodiment, when the user successively sends the electromyographic signals to the terminal, the user can further send the control code to the terminal via the control terminal. Then, the terminal can calculate the average value of the characteristic values of the electromyographic signals. When the user determines that the electromyographic signal currently sent is improper or wrong, the user can further send an electromyographic signal again at a preset time interval. When the electromyographic signal is detected by the electromyographic signal detecting device, and when the control code is not received from the control terminal for a preset time interval, the terminal deletes the detected electromyographic signal. To improve the user experience, the terminal can display a remind information to remind the user to send the electromyographic signal again.

At step S100, when the control code is detected from the control terminal, the terminal associates the obtained characteristic value with the received control code, and stores the obtained characteristic value and the associated control code to the mapping relationship between the characteristic values and the control codes.

In this embodiment, the user can increase a new mapping relationship between the characteristic values and the control codes by inputting the electromyographic signal and the corresponding control code.

For example, the user wants to switch the terminal between different working modes via gestures. However, the standard gestures currently stored in the terminal include no standard gesture for switching the terminal between working modes. The user needs to add the standard gesture for switching the terminal between working modes to the terminal. Adding the standard gesture for switching the terminal between working modes to the terminal includes following steps. The user sends the gesture learning command to the terminal via the control terminal or other ways, and performs the gesture for switching the terminal between working modes. If the gesture is sliding the hand toward a preset direction, the electromyographic signal detecting device sends the electromyographic signal to the terminal. The terminal obtains the characteristic value of the electromyographic signal. The user sends a control code for switching the terminal between working modes to the terminal by operating the corresponding button of the remote controller. The terminal associate the received control code with the obtained characteristic value, that is, the terminal determines the received control code as a control code corresponding to the gesture of sliding the hand toward a preset direction. When the characteristic value received by the terminal is a characteristic value corresponding to the gesture of sliding the hand toward a preset direction, the terminal is switched to another working mode.

What described above are only preferred embodiments of the present disclosure. Accordingly, any equivalent modifications for learning the mapping relationship between the characteristic values and the control codes made on basis of the specification and the attached drawings shall also fall within the scope of the present disclosure.

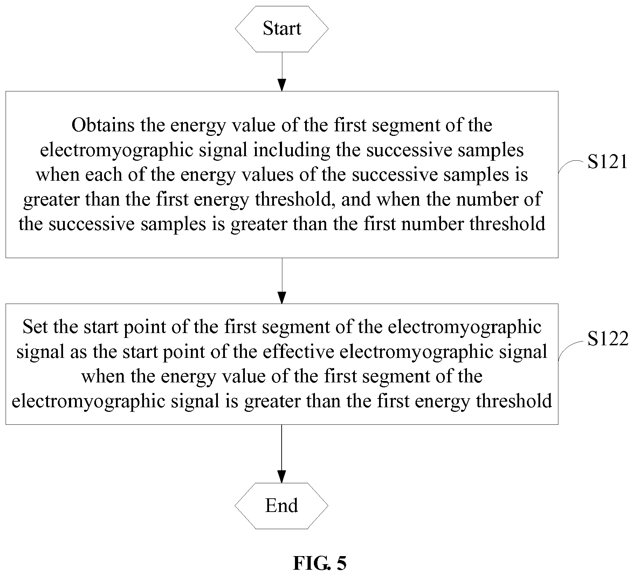

Furthermore, referring to FIG. 5, to improve the accuracy of the characteristic value extraction, the above step S12 further includes following steps.

At step S121, when each of the energy values of a number of successive sample points is greater than the first energy threshold, and when the number of the successive sample points is greater than the first number threshold, the terminal obtains the energy value of the first segment of the electromyographic signal including the successive sample points.

At step S122, when the energy value of the first segment of the electromyographic signal is greater than the first energy threshold, the terminal sets the start point of the first segment of the electromyographic signal as the start point of the effective electromyographic signal.

In this embodiment, the energy value of the first segment of the electromyographic signal is calculated according to a function: $Qn = \int_{tn-T}^{tn+T} x^2(t)dt$. Wherein, Qn denotes the energy value of the first segment of the electromyographic signal, tn denotes an occurring time point of the first segment of the electromyographic signal, T denotes a lasting duration of the first segment of the electromyographic signal, and x denotes an amplitude corresponding to each time point of the first segment of the electromyographic signal. In this embodiment, the energy value of the first segment of the electromyographic signal is calculated to ensure that the detected electromyographic signal starts from the first segment of the electromyographic signal. The first energy threshold is preset, and can be determined by an average value of the start points of the electromyographic signals corresponding to different gestures.

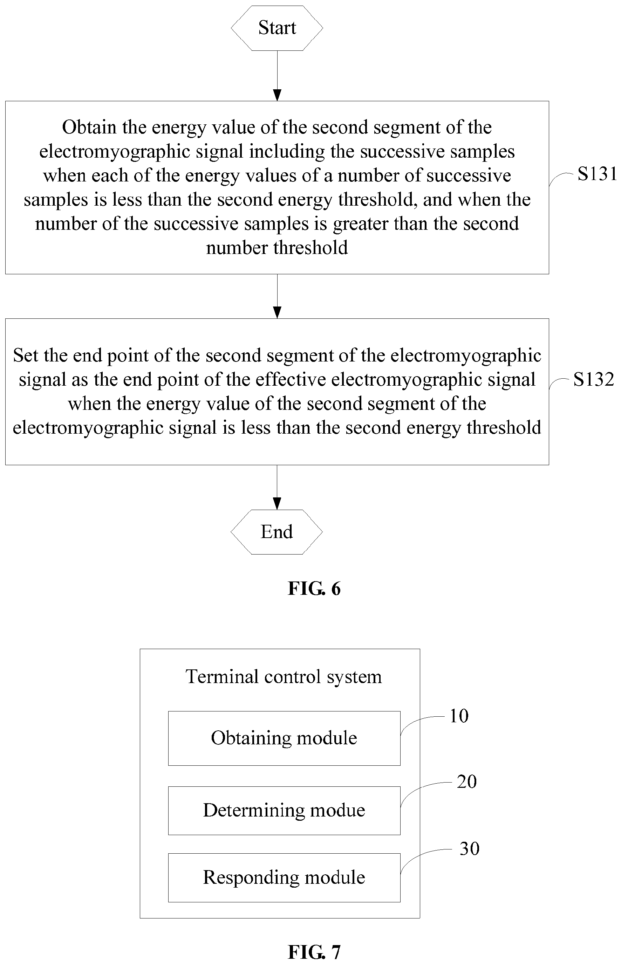

Furthermore, to improve the accuracy of the characteristic value extraction, referring to FIG. 6, the above step S13 further includes following steps.

At step S131, when each of the energy values of a number of successive sample points is less than the second energy threshold, and when the number of the successive sample points is greater than the second number threshold, the terminal obtains the energy value of the second segment of the electromyographic signal including the successive sample points.

At step S132, when the energy value of the second segment of the electromyographic signal is less than the second energy threshold, the terminal sets the end point of the second segment of the electromyographic signal as the end point of the effective electromyographic signal.

In this embodiment, the energy value of the second segment of the electromyographic signal is calculated according to a function: $Qn = \int_{tn-T}^{tn+T} x^2(t)dt$.

Wherein, Qn denotes the energy value of the second segment of the electromyographic signal, tn denotes an occurring time point of the second segment of the electromyographic signal, T denotes a lasting duration of the first segment of the electromyographic signal, and x denotes an amplitude corresponding to each time point of the second segment of the electromyographic signal. In this embodiment, the energy value of the second segment of the electromyographic signal is calculated to ensure that the detected electromyographic signal is ended by the seccond segment of the electromyographic signal. In this embodiment, the second energy value can equal to the first energy value. The second energy threshold is preset, and can be determined by an average value of the end points of the electromyographic signals corresponding to different gestures.

The present disclosure further provides a terminal control system.

FIG.7 illustrates a block diagram of an embodiment of a terminal control system.

For the ordinary skill in the art, the block diagram illustrated by

FIG. 7 is only a preferred embodiment of the present disclosure. Accordingly, equivalent modifications can be made by the ordinary skill in the art on basis of the block diagram illustrated by FIG. 7. Each module of the block diagram is defined to help the technicians to understand the fuction of the modules of the terminal control system, but is not intended to limit the scope of the present disclosure.

The terminal control system includes following modules.

An obtaining module 10 is configured to obtain a characteristic value of the electromyographic signal when the electromyographic signal is detected by an electromyographic signal detecting device. The characteristic value is an average value or a variance of wavelet coefficients obtained by performing wavelet decomposition of the electromyographic signal.

In this embodiment, the electromyographic signal detecting device can be employed inside a wristband or a wristwatch to detect a gesture of a user. When the electromyographic signal detecting device detects the electromyographic signal, the electromyographic signal detecting device sends the detected electromyographic signal to a corresponding terminal.

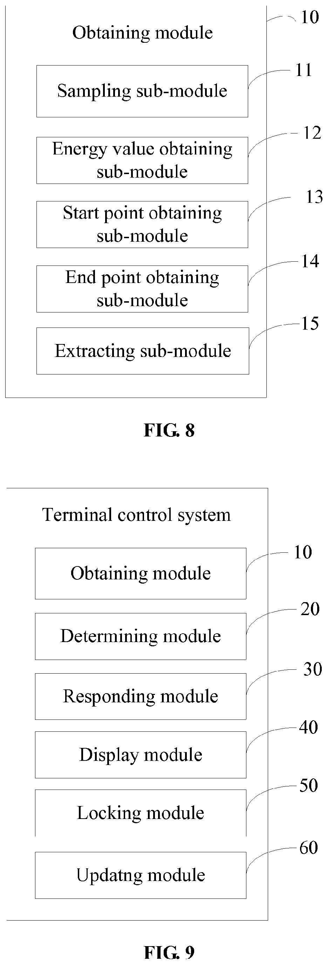

FIG. 8 illustrates that the obtaining module 10 including a number of sub-modules for obtaining the characteristic value of the electromyographic signal detected by the electromyographic signal detecting device.

A sampling sub-module 11 is configured to sample the electromyographic signal to obtain a number of sample points when the electromyographic signal is detected by the electromyographic signal detecting device.

An energy value obtaining sub-module 12 is configured to obtain an energy value of each sample point.

To maintain the accuracy of the obtained electromyographic signal, when the sampling sub-module samles the electromyographic signal, the electromyographic signal can first be filtered and denoised. For example, the frequency of a common electromyographic signal ranges from 20HZ to 500HZ, the detected electromyographic signal can be filtered via a band-pass filter which is used to filter signals with a frequency less than 20HZ and greater than 500HZ. The denoise of the electromyographic signal is well-known.

In this embodiment, the sampling sub-module can sample the detected electromyographic signal by multiple ways. Example a: the electromyographic signal is sampled at each preset time interval until a preset number of sample points are obtained, the preset time interval is determined based on a lasting duration of the electromyographic signal; Example b: the electromyographic signal is sampled at each preset time interval until a preset number of sample points are obtained, and the obtained sample points are further sampled to obtain the final sample points, for example, the electromyographic signal is sampled to obtain one thousand sample points, and the one thousand sample points are further sampled to obtain the final five hundred sample points. What described above are only preferred embodiments of the present disclosure. Any equivalent modifications to sample the electromyographic signal according to need shall also fall within the scope of the present disclosure.

In this embodiment, since the electromyographic signal is presented as a waveform, the energy value of each sample point equals to a square value of the amplitude of a point included in the waveform corresponding to the sample point.

A start point obtaining sub-module 13 is configured to set a start point of a first segment of the electromyographic signal including a number of successive sample points as a start point of an effective electromyographic signal, when each of the energy values of the successive sample points is greater than a first energy threshold, and when the number of the successive sample points is greater than a first number threshold.

In this embodiment, when each of the energy values of the successive sample points is greater than the first energy threshold, the electromyographic signal starts from the first segment of the electromyographic signal. Then, the start point obtaining sub-module 13 sets the start point of the first segment of the electromyographic signal including the successive sample points as the start point of the effective electromyographic signal. The first energy threshold and the first number threshold are preset, and the first energy threshold can be determined by an average value of the energy values of the start points of the electromyographic signals corresponding to different gestures.

An end point obtaining sub-module 14 is configured to set an end point of a second segment of the electromyographic signal including a number of successive sample points as an end point of the effective electromyographic signal, when each of the energy values of the successive sample points is less than a second energy threshold, and when the number of the successive sample points is greater than a second number threshold.

In this embodiment, when each of the energy values of the successive sample points is less than the second energy threshold, the electromyographic signal is ended by the second segment of the electromyographic signal. Then, the end point obtaining sub-module 14 sets the end point of the second segment of the electromyographic signal including the successive sample points as the end point of the effective electromyographic signal. The second energy threshold and the second number threshold are preset, and the second energy threshold can be determined by an average value of the energy values of the end points of the electromyographic signals corresponding to different gestures. The second energy value can equal to the first energy value, and can also be different from the first energy value.

The extracting sub-module 15 is configured to extract the characteristic value of the effective electromyographic signal between the start point and the end point.

When the extracting sub-module 15 extracts the characteristic value, the extracting sub-module 15 can first perform the electromyographic signal by wavelet decomposition, and calculate an average value or a variance of wavelet coefficients to obtain the characteristic value. The calculation of the wavelet coefficient and the variance thereof is well-known.

A determining module 20 is configured to search the mapping relationship between preset characteristic values and control codes to determine whether there is a preset characteristic value which can match the obtained characteristic value, if yes, the terminal determining module 20 is configured to determine a control code corresponding to the matching preset characteristic value.

In this embodiment, since the wristband or the wristwatch employing the electromyographic signal may move when the gesture is performed by the user, the detected electromyographic signal may fluctuate. Thus, when there is a preset characteristic value which can match the obtained characteristic value, a difference between the preset characteristic value and the obtained characteristic value is less than a preset threshold.

Furthermore, the electromyographic signals corresponding to a same gesture performed by different users may be different, that is, the preset characteristic values and control codes of different users may be different. To improve the flexibility of the terminal control, the determining module 20 further includes following units. An obtaining unit is configured to determine the identifier of the electromyographic signal detecting device according to the obtained electromyographic signal, obtain a mapping relationship between a preset characteristic value corresponding to the identifier and the control code according to the mapping relationship between the preset characteristic values and the control codes and according to an association between the identifiers. A determining unit is configured to determine a control code corresponding to the matching preset characteristic value, when a characteristic value matches the preset characteristic value in the mapping relationship between the preset characteristic values and the control codes.

A responding module 30 is configured to respond to a control signal corresponding to the determined control code.

In the terminal control system of the present disclosure, when the electromyographic signal is detected by the electromyographic signal detecting device, the obtaining module 10 can obtain the characteristic value of the detected electromyographic signal, and determines the control code corresponding to the obtained characteristic value. The responding module 30 responds to the determined control code, thereby allowing the terminal to be controlled by a gesture. The electromyographic signal detecting device has no need to employ multiple unnecessary mechanical buttons, but can send the electromyographic signal based on the gesture of the user, thereby decreasing the size thereof and making it portable and flexible to be controlled.

In the terminal control system of the present disclosure, the mapping relationship between the preset characteristic values and the control codes is preset as follows.

The sampling sub-module 11 collects the electromyographic signal, preprocesses the collected electromyographic signal, and samples the preprocessed electromyographic signal to obtain a number of sample points. The energy value obtaining sub-module 12 obtains the energy value of each sample point. When each of the energy values of the successive sample points is greater than the first energy threshold, and when the number of the successive sample points is greater than the first number threshold, the energy value obtaining sub-module 12 sets the start point of the first segment of the electromyographic signal as the start point of the effective electromyographic signal. When each of the energy values of the successive sample points is less than the second energy threshold, and when the number of the successive sample points is greater than the second number threshold, the end point obtaining sub-module 14 sets the end point of the second segment of the electromyographic signal including the successive sample points as the end point of the effective electromyographic signal. The extracting sub-module 15 extracts the characteristic value of the effective electromyographic signal between the start point and the end point. Then, the obtained characteristic value is associated with the control code, the obtained characteristic value and the associated control code is stored, and the mapping relationship between the characteristic values and the control codes is generated. In this embodiment, the mapping relationship is generated based on a training pattern classifier. The generation of the mapping relationship by the training pattern classifier is well-known.

To improve the accuracy and flexibility of the terminal control, the terminal control system can further learn the mapping relationship between the preset characteristic values and the control codes.

FIG. 9 illustrates that a first embodiment of the terminal control system which can learn the mapping relationship between the preset characteristic values and the control codes.

A display module 40 is configured to display a selecting interface including a number of standard gesture pictures when a gesture learning command is detected, for a user to select a standard gesture picture, and trigger a selecting command after the standard gesture picture is selected.

In this embodiment, when terminal fails in detecting the gesture learning command, the user can send the gesture learning command to the terminal by a control terminal (such as a remote controller of a smart phone) or a voice control command. In other embodiments, when the terminal detects a same electromyographic signal for a number of times within a preset time interval and the number of times is greater than a preset threshold, the terminal determines that it fails in detecting the gesture learning command and triggers the gesture learning command.

A locking module 50 is configured to lock the terminal into displaying the corresponding standard gesture picture according to the selecting command, when the selecting command is received.

The obtaining module 10 is further configured to obtain the characteristic value of the electromyographic signal when the electromyographic signal is detected by the electromyographic signal detecting device.

When the terminal obtains the characteristic value of the detected electromyographic signal, the obtaining module 10 can first filter and denoise the electromyographic signal according to the first embodiment, determines the start point and the end point of the electromyographic signal, treats the electromyographic signal between the start point and the end point as the effective electromyographic signal, and obtains the characteristic value of the effective electromyographic signal.

To improve the accuracy of the learning of the mapping relationship, when the electromyographic signal is detected by the electromyographic signal detecting device, the obtaining module 10 displays a confirming interface. When a confirming command is detected by the confirming interface, the obtaining module 10 obtains the characteristic value of the obtained electromyographic signal.

An updating module 60 is configured to determine the control code corresponding to the standard gesture picture currently displayed, and update the characteristic value corresponding to the determined control code to the obtained characteristic value.

In this embodiment, the user can send a number of electromyographic signals within a preset time interval. When the terminal detects a number of electromyographic signals within a preset time interval, the obtaining module 10 can obtains an average value of the characteristic values of the electromyographic signals, and updates the control code corresponding to the obtained characteristic value according to the obtained average value.

To improve the accuracy of the terminal control, when the gesture learning command is detected, the display module 40 can successively displays the standard gesture pictures. After each standard gesture picture is displayed, the display module 40 detects the electromyographic signal. When the electromyographic signal is detected, the determining module 20 determines a control code corresponding to the standard gesture picture currently displayed. The updating module 60 updates the characteristic value corresponding to the determined control code to the obtained characteristic value. The display module 40 displays a next standard gesture picture and continues to learn the mapping relationship. Since different users have different habits to perform a same standard gesture, the standard gesture performed may thus be imperfect or inaccurate. For example, the user may perform a gesture according to his or her habit by placing the palm upwards about 45 degrees. However, a preset standard gesture needs the user to place the palm upwards about 75 degrees, which may make the gesture performed by the user difficult to be identified. Thus, the user may need to learn the standard gesture according to following steps. The user sends a gesture learning command to the terminal via a control terminal for example. When the terminal receives the gesture learning command, the terminal displays a standard gesture picture interface. The user can select a standard gesture picture of the palm placing upwards 75 degrees via the standard gesture picture interface, and performs a gesture according to his or her habit (for example, the user place the palm upwards about 45 degrees). Then, the electromyographic signal detecting device sends the electromyographic signal to the terminal. The terminal obtains the characteristic value of the electromyographic signal, determines a control code corresponding to the standard gesture picture of the palm placing upwards 75 degrees, and updates the characteristic value corresponding to the determined control code to the obtained characteristic value corresponding to the standard gesture picture of the palm placing upwards about 45 degrees.

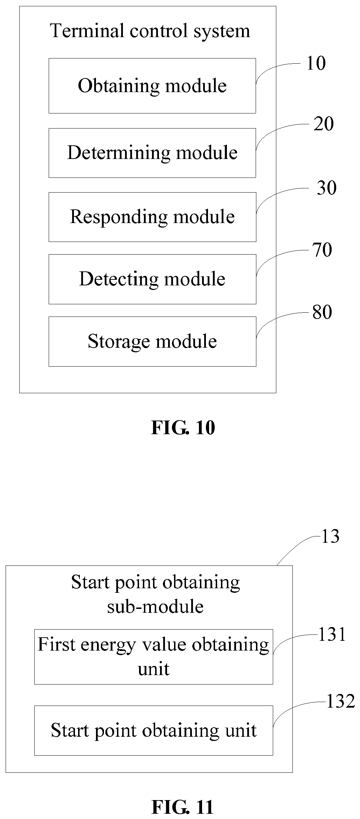

FIG. 10 illustrates a second embodiment of the terminal control system which an learn the mapping relationship between the preset characteristic values and the control codes.

The detecting module 70 is configured to detect the electromyographic signal from the electromyographic signal detecting device when the gesture learning command is detected.

In this embodiment, when terminal fails in detecting the gesture learning command, the user can trigger the gesture learning command by a control terminal (such as a remote controller of a smart phone) or a voice control command. In other embodiments, when the terminal detects a same electromyographic signal for a number of times within a preset time interval and the number of times is greater than a preset threshold, the terminal determines that it fails in detecting the gesture learning command and triggers the gesture learning command.

The obtaining module 10 is configured to obtain the characteristic value of the electromyographic signal when the electromyographic signal is detected by the electromyographic signal detecting device, and receive the control code from the control terminal.

In this embodiment, when the user successively sends the electromyographic signals to the terminal, the user can further send the control code to the terminal via the control terminal. Then, the terminal can calculate the average value of the characteristic values of the electromyographic signals. When the user determines that the electromyographic signal currently sent is improper or wrong, the user can further sends an electromyographic signal again at a preset time interval. When the electromyographic signal is detected by the electromyographic signal detecting device, and when the control code is not received from the control terminal for a preset time interval, the terminal deletes the detected electromyographic signal. To improve the user experience, the display module 40 can display a remind information to remind the user to send the electromyographic signal again.

The storage module 80 is configured to associate the obtained characteristic value with the received control code and stores the obtained characteristic value and the associated control code to the mapping relationship between the characteristic values and the control codes, when the control code is detected by the control code.

In this embodiment, the user can increase a new mapping relationship between the characteristic values and the control codes by inputting the electromyographic signal and the corresponding control code.

For example, the user wants to switch the terminal between different working modes via gestures. However, the standard gestures currently stored in the terminal include no standard gesture for switching the terminal between working modes. The user needs to add the standard gesture for switching the terminal between working modes to the terminal. Adding the standard gesture for switching the terminal between working modes to the terminal includes following steps. The user sends the gesture learning command to the terinal via the control terminal or other ways, and performs the gesture for switching the terminal between working modes. If the gesture is sliding the hand toward a preset direction, the electromyographic signal detecting device sends the electromyographic signal to the terminal. The terminal obtains the characteristic value of the electromyographic signal. The user sends a control code for switching the terminal between working modes to the terminal by operating the corresponding button of the remote controller. The terminal associate the received control code with the obtained characteristic value, that is, the terminal determines the received control code as a control code corresponding to the gesture of sliding the hand toward a preset direction. When the characteristic value received by the terminal is a characteristic value corresponding to the gesture of sliding the hand toward a preset direction, the terminal is switched to another working mode.

What described above are only preferred embodiments of the present disclosure. Accordingly, any equivalent modifications for learning the mapping relationship between the characteristic values and the control codes made on basis of the specification and the attached drawings shall also fall within the scope of the present disclosure.

To improve the accuracy of the characteristic value extraction, referring to FIG. 11, the start point obtaining sub-module 13 further includes following units.

A first energy value obtaining unit 131 is configured to obtain the energy value of the first segment of the electromyographic signal including the successive sample points, when each of the energy values of the successive sample points is greater than the first energy threshold, and when the number of the successive sample points is greater than the first number threshold.

A start point obtaining unit 132 is configured to set the start point of the first segment of the electromyographic signal as the start point of the effective electromyographic signal, when the energy value of the first segment of the electromyographic signal is greater than the first energy threshold.

In this embodiment, the energy value of the first segment of the electromyographic signal is calculated according to a function: $Qn = \int_{tn-T}^{tn+T} x^2(t)dt$. Wherein, Qn denotes the energy value of the first segment of the electromyographic signal, tn denotes an occurring time point of the first segment of the electromyographic signal, T denotes a lasting duration of the first segment of the electromyographic signal, and x denotes an amplitude corresponding to each time point of the first segment of the electromyographic signal. In this embodiment, the energy value of the first segment of the electromyographic signal is calculated to ensure that the detected electromyographic signal starts from the first segment of the electromyographic signal. The first energy threshold is preset, and can be determined by an average value of the start points of the electromyographic signals corresponding to different gestures.

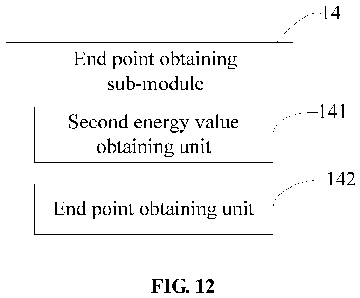

Furthermore, to improve the accuracy of the characteristic value extraction, referring to FIG. 12, the end point obtaining sub-module 14 further includes following units.

A second energy value obtaining unit 141 is configured to obtain the energy value of the second segment of the electromyographic signal including the successive sample points, when each of the energy values of the successive sample points is less than the second energy threshold, and when the number of the successive sample points is greater than the second number threshold.

An end point obtaining unit 142 is configured to set the end point of the second segment of the electromyographic signal as the end point of the effective electromyographic signal, when the energy value of the second segment of the electromyographic signal is less than the second energy threshold.

In this embodiment, the energy value of the second segment of the electromyographic signal is calculated according to a function: $Qn = \int_{tn-T}^{tn+T} x^2(t)dt$.

Wherein, Qn denotes the energy value of the second segment of the electromyographic signal, tn denotes an occurring time point of the second segment of the electromyographic signal, T denotes a lasting duration of the first segment of the electromyographic signal, and x denotes an amplitude corresponding to each time point of the second segment of the electromyographic signal. In this embodiment, the energy value of the second segment of the electromyographic signal is calculated to ensure that the detected electromyographic signal is ended by the seccond segment of the electromyographic signal. In this embodiment, the second energy value can equal to the first energy value. The second energy threshold is preset, and can be determined by an average value of the end points of the electromyographic signals corresponding to different gestures.

It is notable that in this present disclosure, the terms "including", "comprise", or any other variants are non-exclusive, which makes a process, method, object, or system including a series of elements to include not only those elements, but also other elements which are not be clearly listed or other elements inherently included in the process, method, object, or system.

The order of the above embodiments is just used for better describing the embodiments, but does not represents which one is better.

Through the above described embodiments, those persons skilled in the art may clearly understand that the above-described embodiments may be implemented by software combined with a necessary universal hardware platform, of course, may also be implemented only by hardware. However, in many cases, the former is a better embodiment. Based on this understanding, the present disclosure, particularly the part of it which makes a contribution to the prior art, may be presented as a software product. The computer software product is stored in a storage medium (such as ROM/RAM, disk, CD), including a number of instructions to instruct a terminal device (may be a cell phone, a computer, a server, or a network equipment) to perform the embodiments described in the present disclosure.

What described above are only preferred embodiments of the present disclosure but are not intended to limit the scope of the present disclosure. Accordingly, any equivalent structural or process flow modifications that are made on basis of the specification and the attached drawings or any direct or indirect applications in other technical fields shall also fall within the scope of the present disclosure.

**Claims**

1. A terminal control method comprising:

> a terminal obtaining (S10) a characteristic value of an electromyographic signal when the electromyographic signal is detected by an electromyographic signal detecting device, the characteristic value being an average value or a variance of wavelet coefficients obtained by performing wavelet decomposition of the electromyographic signal;
> the terminal searching (S20) a mapping relationship between preset characteristic values and control codes to determine whether there is a preset characteristic value which can match the obtained characteristic value, and determining a control code corresponding to the matching preset characteristic value if there is a matching preset characteristic value; and
> the terminal responding (S30) to a control signal corresponding to the determined control code, wherein the step of "the terminal obtaining (10) a characteristic value of an electromyographic signal when the electromyographic signal is detected by an electromyographic signal detecting device" further comprises:

>> sampling (S11) the electromyographic signal to obtain a plurality of sample points and obtaining an energy value of each sample point when the electromyographic signal is detected by the electromyographic signal detecting device;
>> setting (S12) a start point of a first segment of the electromyographic signal including a plurality of successive sample points as a start point of an effective electromyographic signal when each of the energy values of the successive sample points is greater than a first energy threshold, and when the number of the successive sample points is greater than a first number threshold;
>> setting (S13) an end point of a second segment of the electromyographic signal including a plurality of successive sample points as an end point of the effective electromyographic signal when each of the energy values of the successive sample points is less than a second energy threshold, and when the number of the successive sample points included in the second segment is greater than a second number threshold; and

extracting (S14) the characteristic value of the effective electromyographic signal between the start point and the end point.

2. The terminal control method of claim 1, wherein the step of "setting (S12) a start point of a first segment of the electromyographic signal including a plurality of successive sample points as a start point of an effective electromyographic signal when each of the energy values of the successive sample points is greater than a first energy threshold, and when the number of the successive sample points is greater than a first number threshold" further comprises:

obtaining (S121) the energy value of the first segment of the electromyographic signal including the successive sample points when each of the energy values of the successive sample points is greater than the first energy threshold, and when the number of the successive sample points is greater than the first number threshold; and

setting (S122) the start point of the first segment of the electromyographic signal as the start point of the effective electromyographic signal when the energy value of the first segment of the electromyographic signal is greater than the first energy threshold.

3. The terminal control method of claim 1, wherein the step of "setting (S13) an end point of a second segment of the electromyographic signal including the successive sample points as an end point of the effective electromyographic signal when each of the energy values of the successive sample points is less than a second energy threshold, and when the number of the successive sample points is greater than a second number threshold" further comprises:

obtaining (S131) the energy value of the second segment of the electromyographic signal including the successive sample points when each of the energy values of the successive sample points is less than the second energy threshold, and when the number of the successive sample points is greater than the second number threshold; and

setting (S132) the end point of the second segment of the electromyographic signal as the end point of the effective electromyographic signal when the energy value of the second segment of the electromyographic signal is less than the second energy threshold.

4. The terminal control method of claim 2, wherein the step of "setting (S13) an end point of a second segment of the electromyographic signal including the successive sample points as an end point of the effective electromyographic signal when each of the energy values of the successive sample points is less than a second energy threshold, and when the number of the successive sample points is greater than a second number threshold" further comprises:

obtaining (S131) the energy value of the second segment of the electromyographic signal including the successive sample points when each of the energy values of the successive sample points is less than the second energy threshold, and when the number of the successive sample points is greater than the second number threshold; and

setting (S132) the end point of the second segment of the electromyographic signal as the end point of the effective electromyographic signal when the energy value of the second segment of the electromyographic signal is less than the second energy threshold.

5. The terminal control method of claim 1, further comprising learning the mapping relationship between the preset characteristic values and the control codes comprising:

displaying (S40) a selecting interface including a plurality of standard gesture pictures when a gesture learning command is detected, for a user to select a standard gesture picture, and triggering a selecting command after the standard gesture picture is selected;
locking (S50) the terminal into displaying the corresponding standard gesture picture according to the selecting command when the selecting command is received;
obtaining (S60) the characteristic value of the electromyographic signal when the electromyographic signal is detected by the electromyographic signal detecting device; and
determining (S70) the control code corresponding to the standard gesture picture currently displayed, and updating the characteristic value corresponding to the determined control code to the obtained characteristic value.

6. The terminal control method of claim 1, further comprising learning the mapping relationship between the preset characteristic values and the control codes, comprising:

detecting (S80) the electromyographic signal from the electromyographic signal detecting device when the gesture learning command is detected;

obtaining (S90) the characteristic value of the electromyographic signal, and receiving the control code from the control terminal when the electromyographic signal is detected by the electromyographic signal detecting device; and

associating (S100) the obtained characteristic value with the received control code when the control code is detected from the control terminal, and storing the obtained characteristic value and the associated control code to the mapping relationship between the characteristic values and the control codes.

7. The terminal control method of claim 6, wherein when the gesture learning command is detected, the method further comprises:

triggering the gesture learning command when the terminal detects a same electromyographic signal for a number of times within a preset time interval and the number of times is greater than a preset threshold.

8. The terminal control method of claim 1, further comprising learning the mapping relationship between the preset characteristic values and the control codes comprising:

successively displaying the standard gesture pictures when the gesture learning command is detected;

detecting the electromyographic signal after each standard gesture picture is displayed;

determining the control code corresponding to the standard gesture picture currently displayed when the electromyographic signal is detected;

updating the characteristic value corresponding to the determined control code to the obtained characteristic value; and

displaying a next standard gesture picture and continue to learn the mapping relationship.

9. The terminal control method of claim 1, wherein the step of "the terminal searching (S20) a mapping relationship between preset characteristic values and control codes to determine whether there is a preset characteristic value which can match the obtained characteristic value, and determining a control code corresponding to the matching preset characteristic value if there is a matching preset characteristic value" further comprises:

determining an identifier of the electromyographic signal detecting device according to the detected electromyographic signal;

obtaining a mapping relationship between a preset characteristic value corresponding to the identifier and the control code, according to the mapping relationship between the preset characteristic values and the control codes, and according to an association between the identifiers; and

determining the control code corresponding to the matching preset characteristic value, when a characteristic value matches the preset characteristic value in the mapping relationship between the preset characteristic values and the control codes.

10. A terminal control system comprising:

an obtaining module (10) configured to obtain a characteristic value of an electromyographic signal when the electromyographic signal is detected by an electromyographic signal detecting device, the characteristic value being an average value or a variance of wavelet coefficients obtained by performing wavelet decomposition of the electromyographic signal; and

a determining module (20) configured to search a mapping relationship between preset characteristic values and control codes to determine whether there is a preset characteristic value which can match the obtained characteristic value, and determining a control code corresponding to the matching preset characteristic value if there is a matching preset characteristic value; and

a responding module (30) configured to respond to a control signal corresponding to the determined control code, wherein the obtaining module (10) comprises:

a sampling sub-module (11) configured to sample the electromyographic signal to obtain a plurality of sample points and obtains an energy value of each sample point;

an energy value obtaining sub-module (12) configured to obtain an energy value of each sample point when the electromyographic signal is detected by the electromyographic signal detecting device;

a start point obtaining sub-module (13) configured to set a start point of a first segment of the electromyographic signal including a plurality of successive sample points as a start point of an effective electromyographic signal when each of the energy values of the successive sample points is greater than a first energy threshold, and when the number of the successive sample points is greater than a first number threshold; an end point obtaining sub-module (14) configured to set an end point of a second segment of the electromyographic signal including a plurality of successive sample points as an end point of the effective electromyographic signal when each of the energy values of the successive sample points is less than a second energy threshold, and when the number of the successive sample points included in the second segment is greater than a second number threshold; and

an extracting sub-module (15) configured to extract the characteristic value of the effective electromyographic signal between the start point and the end point.

11. The terminal control system of claim 10, wherein the start point obtaining sub-module (13) further comprises:

a first energy value (131) obtaining unit configured to obtain the energy value of the first segment of the electromyographic signal including the successive samples when each of the energy values of the successive samples is greater than the first energy threshold, and when the number of the successive samples is greater than the first number threshold; and a start point obtaining unit (132) configured to set the start point of the first segment of the electromyographic signal as the start point of the effective electromyographic signal when the energy value of the first segment of the electromyographic signal is greater than the first energy threshold.

12. The terminal control system of claim 10, wherein the end point obtaining sub-module (14) further comprises:

a second energy value obtaining unit (141) configured to obtain the energy value of the second segment of the electromyographic signal including the successive sample points when each of the energy values of the successive sample points is less than the second energy threshold, and when the number of the successive sample points is greater than the second number threshold; and an end point obtaining unit (142) configured to set the end point of the second segment of the electromyographic signal as the end point of the effective electromyographic signal when the energy value of the second segment of the electromyographic signal is less than the second energy threshold.

13. The terminal control system of claim 11, wherein the end point obtaining sub-module (14) further comprises:

a second energy value obtaining unit (141) configured to obtain the energy value of the second segment of the electromyographic signal including the successive sample points when each of the energy values of the successive sample points is less than the second energy threshold, and when the number of the successive sample points is greater than the second number threshold; and an end point obtaining unit (142) configured to set the end point of the second segment of the electromyographic signal as the end point of the effective electromyographic signal when the energy value of the second segment of the electromyographic signal is less than the second energy threshold.

14. The terminal control system of claim 10, further comprising:

a display module (40) configured to display a selecting interface including a plurality of standard gesture pictures when a gesture learning command is detected, for a user to select a standard gesture picture, and trigger a selecting command after the standard gesture picture is selected; a locking module (50) configured to lock the terminal into displaying the corresponding standard gesture picture according to the selecting command when the selecting command is received; an obtaining module (10) configured to obtain the characteristic value of the electromyographic signal when the electromyographic signal is detected by the electromyographic signal detecting device; and an updating module (60) configured to determine the control code corresponding to the standard gesture picture currently displayed, and update the characteristic value corresponding to the determined control code to the obtained characteristic value.

15. The terminal control system of claim 10, further comprising:

a detecting module (70) configured to detect the electromyographic signal from the electromyographic signal detecting device when the gesture learning command is detected; and a storage module (80); wherein the obtaining module (10) is further configured to obtain the characteristic value of the electromyographic signal, and receive the control code from the control terminal when the electromyographic signal is detected by the electromyographic signal detecting device;

the storage module (80) is configured to associate the obtained characteristic value with the received control code when the control code is detected by the control code, and store the obtained characteristic value and the associated control code to the mapping relationship between the characteristic values and the control codes.

16. The terminal control system of claim 10, further comprising:

a display module (40) configured to successively display the standard gesture pictures when the gesture learning command is detected;
a detecting module (70) configured to detect the electromyographic signal after each standard gesture picture is displayed;
a determining module (20) configured to determine the control code corresponding to the standard gesture picture currently displayed when the electromyographic signal is detected; and
an updating module (60) configured to update the characteristic value corresponding to the determined control code to the obtained characteristic value;

wherein the display module (40) is further configured to display a next standard gesture picture and continue to learn the mapping relationship.

17. The terminal control system of claim 10, wherein the determining module (20) further comprises:

an obtaining unit configured to determine an identifier of the electromyographic signal detecting device according to the detected electromyographic signal, and obtain a mapping relationship between a preset characteristic value corresponding to the identifier and the control code, according to the mapping relationship between the preset characteristic values and the control codes, and according to an association between the identifiers; and
a determining unit configured to determine the control code corresponding to the matching preset characteristic value, when a characteristic value matches the preset characteristic value in the mapping relationship between the preset characteristic values and the control codes.

**Patentansprüche**

1. Endgerätsteuerverfahren, aufweisend:

Erhalten (S10) eines Kennwerts eines elektromyographischen Signals durch ein Endgerät, wenn das elektromyographische Signal durch eine Erfassungsvorrichtung für elektromyographische Signale erfasst wird, wobei der Kennwert ein Durchschnittswert oder eine Varianz von Wavelet-Koeffizienten ist,
die durch Durchführen einer Wavelet-Zerlegung des elektromyographischen Signals erhalten werden;
Suchen (S20) eines Abbildungsverhältnisses zwischen voreingestellten Kennwerten und Steuercodes durch das Endgerät, um zu bestimmen, ob es einen voreingestellten Kennwert gibt, der zu dem erhaltenen Kennwert passen kann, und Bestimmen eines Steuercodes, der dem passenden voreingestellten Kennwert entspricht, falls es einen passenden voreingestellten Kennwert gibt; und
Antworten (S30) des Endgeräts auf ein Steuersignal, das dem bestimmten Steuercode entspricht, wobei der Schritt des "Erhalten (S10) eines Kennwerts eines elektromyographischen Signals durch ein Endgerät, wenn das elektromyographische Signal durch eine Erfassungsvorrichtung für elektromyographische Signale erfasst wird" ferner aufweist:

Abtasten (S11) des elektromyographischen Signals, um eine Mehrzahl an Abtastpunkten zu erhalten und Erhalten eines Energiewerts jedes Abtastpunkts, wenn das elektromyographische Signal durch die Erfassungsvorrichtung für elektromyographische Signale erfasst wird;
Einstellen (S12) eines Startpunkts eines ersten Segments des elektromyographischen Signals, das eine Mehrzahl an aufeinanderfolgenden Abtastpunkten als einen Startpunkt eines effektiven elektromyographischen Signals beinhaltet, wenn jeder der Energiewerte der aufeinanderfolgenden Abtastpunkte größer ist als eine erste Energieschwelle, und wenn die Anzahl der aufeinanderfolgenden Abtastpunkte größer ist als eine erste Anzahlschwelle;
Einstellen (S13) eines Endpunkts eines zweiten Segments des elektromyographischen Signals, das eine Mehrzahl an aufeinanderfolgenden Abtastpunkten als einen Endpunkt des effektiven elektromyographischen Signals beinhaltet, wenn jeder der Energiewerte der aufeinanderfolgenden Abtastpunkte geringer ist als eine zweite Energieschwelle, und wenn die Anzahl der aufeinanderfolgenden Abtastpunkte, die in dem zweiten Segment enthalten sind, größer ist als eine zweite Anzahlschwelle; und

Extrahieren (S14) des Kennwerts des effektiven elektromyographischen Signals zwischen dem Startpunkt und dem Endpunkt.

2. Endgerätesteuerverfahren nach Anspruch 1, wobei der Schritt des "Einstellens (S12) eines Startpunkts eines ersten Segments des elektromyographischen Signals, das eine Mehrzahl an aufeinanderfolgenden Abtastpunkten als einen Startpunkt eines effektiven elektromyographischen Signals beinhaltet, wenn jeder der Energiewerte der aufeinanderfolgenden Abtastpunkte größer ist als eine erste Energieschwelle, und wenn die Anzahl der aufeinanderfolgenden Abtastpunkte größer ist als eine erste Anzahlschwelle" ferner aufweist:

Erhalten (S121) des Energiewerts des ersten Segments des elektromyographischen Signals, das die aufeinanderfolgenden Abtastpunkte beinhaltet, wenn jeder der Energiewerte der aufeinanderfolgenden Abtastpunkte größer ist als die erste Energieschwelle, und wenn die Anzahl der aufeinanderfolgenden Abtastpunkte größer ist als die erste Anzahlschwelle; und

Einstellen (S122) des Startpunkts des ersten Segments des elektromyographischen Signals als den Startpunkt des effektiven elektromyographischen Signals, wenn der Energiewert des ersten Segments des elektromyographischen Signals größer ist als die erste Energieschwelle.

3. Endgerätesteuerverfahren nach Anspruch 1, wobei der Schritt des "Einstellens (S13) eines Endpunkts eines zweiten Segments des elektromyographischen Signals, das eine Mehrzahl an aufeinanderfolgenden Abtastpunkten als einen Endpunkt des effektiven elektromyographischen Signals beinhaltet, wenn jeder der Energiewerte der aufeinanderfolgenden Abtastpunkte geringer ist als eine zweite Energieschwelle, und wenn die Anzahl der aufeinanderfolgenden Abtastpunkte, die in dem zweiten Segment enthalten sind, größer ist als eine zweite Anzahlschwelle" ferner aufweist:

Erhalten (S131) des Energiewerts des zweiten Segments des elektromyographischen Signals, das die aufeinanderfolgenden Abtastpunkte beinhaltet, wenn jeder der Energiewerte der aufeinanderfolgenden Abtastpunkte geringer ist als die zweite Energieschwelle, und wenn die Anzahl der aufeinanderfolgenden Abtastpunkte größer ist als die zweite Anzahlschwelle; und

Einstellen (S132) des Endpunkts des zweiten Segments des elektromyographischen Signals als den Endpunkt des effektiven elektromyographischen Signals, wenn der Energiewert des zweiten Segments des elektromyographischen Signals geringer ist als die zweite Energieschwelle.

4. Endgerätesteuerverfahren nach Anspruch 2, wobei der Schritt des "Einstellens (S13) eines Endpunkts eines zweiten Segments des elektromyographischen Signals, das eine Mehrzahl an aufeinanderfolgenden Abtastpunkten als einen Endpunkt des effektiven elektromyographischen Signals beinhaltet, wenn jeder der Energiewerte der aufeinanderfolgenden Abtastpunkte geringer ist als eine zweite Energieschwelle, und wenn die Anzahl der aufeinanderfolgenden Abtastpunkte, die in dem zweiten Segment enthalten sind, größer ist als eine zweite Anzahlschwelle" ferner aufweist:

Erhalten (S131) des Energiewerts des zweiten Segments des elektromyographischen Signals, das die aufeinanderfolgenden Abtastpunkte beinhaltet, wenn jeder der Energiewerte der aufeinanderfolgenden Abtastpunkte geringer ist als die zweite Energieschwelle, und wenn die Anzahl der aufeinanderfolgenden Abtastpunkte größer ist als die zweite Anzahlschwelle; und

Einstellen (S132) des Endpunkts des zweiten Segments des elektromyographischen Signals als den Endpunkt des effektiven elektromyographischen Signals, wenn der Energiewert des zweiten Segments des elektromyographischen Signals geringer ist als die zweite Energieschwelle.

5. Endgerätesteuerverfahren nach Anspruch 1, ferner aufweisend ein Lernen des Abbildungsverhältnisses zwischen den voreingestellten Kennwerten und den Steuercodes, aufweisend:

Anzeigen (S40) einer Auswahlschnittstelle, die eine Mehrzahl an Standardgestenbildern beinhaltet, wenn ein Gestenlernbefehl erfasst wird, so dass ein Benutzer ein Standardgestenbild auswählt und einen Auswahlbefehl auslöst, nachdem das Standardgestenbild ausgewählt wurde;

Sperren (S50) des Endgeräts, so dass es das entsprechende Standardgestenbild gemäß dem Auswahlbefehl darstellt, wenn der Auswahlbefehl empfangen wird;

Erhalten (S60) des Kennwerts des elektromyographischen Signals, wenn das elektromyographische Signal durch die Erfassungsvorrichtung für elektromyographische Signale erfasst wird; und

Bestimmen (S70) des Steuercodes, der dem Standardgestenbild entspricht, das gegenwärtig dargestellt wird, und Aktualisieren des Kennwerts, der dem bestimmten Steuercode entspricht, auf den erhaltenen Kennwert.

6. Endgerätesteuerverfahren nach Anspruch 1, ferner aufweisend ein Lernen des Abbildungsverhältnisses zwischen den voreingestellten Kennwerten und den Steuercodes, aufweisend:

   Erfassen (S80) des elektromyographischen Signals von der Erfassungsvorrichtung für elektromyographische Signale, wenn der Gestenlernbefehl erfasst wird;
   Erhalten (S90) des Kennwerts des elektromyographischen Signals, und Empfangen des Steuercodes von dem Steuergerät, wenn das elektromyographische Signal durch die Erfassungsvorrichtung für elektromyographische Signale erfasst wird; und
   Zuordnen (S100) des erhaltenen Kennwerts zu dem empfangenen Steuercode, wenn der Steuercode von dem Steuergerät erfasst wird, und Speichern des erhaltenen Kennwerts und des zugeordneten Steuercodes bei dem Abbildungsverhältnis zwischen den Kennwerten und den Steuercodes.

7. Endgerätesteuerverfahren nach Anspruch 6, wobei, wenn der Gestenlernbefehl erfasst wird, das Verfahren ferner aufweist:

   Auslösen des Gestenlernbefehls, wenn das Endgerät ein identisches elektromyographisches Signal eine Anzahl Male innerhalt einer voreingestellten Zeitspanne erfasst und die Anzahl Male größer ist als eine voreingestellte Schwelle.

8. Endgerätesteuerverfahren nach Anspruch 1, ferner aufweisend ein Lernen des Abbildungsverhältnisses zwischen den voreingestellten Kennwerten und den Steuercodes, aufweisend:

   sukzessives Darstellen der Standardgestenbilder, wenn der Gestenlernbefehl erfasst wird;
   Erfassen des elektromyographischen Signals, nachdem jedes Standardgestenbild dargestellt wird;
   Bestimmen des Steuercodes, der dem Standardgestenbild entspricht, das gegenwärtig dargestellt wird, wenn das elektromyographische Signal erfasst wird;
   Aktualisieren des Kennwerts, der dem bestimmten Steuercode entspricht, auf den erhaltenen Kennwert; und
   Anzeigen eines nächsten Standardgestenbilds und fortgesetztes Lernen des Abbildungsverhältnisses.

9. Endgerätesteuerverfahren nach Anspruch 1, wobei der Schritt des "Suchens (S20) eines Abbildungsverhältnisses zwischen voreingestellten Kennwerten und Steuercodes durch das Endgerät, um zu bestimmen, ob es einen voreingestellten Kennwert gibt, der zu dem erhaltenen Kennwert passen kann, und Bestimmen eines Steuercodes, der dem passenden voreingestellten Kennwert entspricht, falls es einen passenden voreingestellten Kennwert gibt" ferner aufweist:

   Bestimmen eines Identifikators der Erfassungsvorrichtung für elektromyographische Signale gemäß dem erfassten elektromyographischen Signal;
   Erhalten eines Abbildungsverhältnisses zwischen einem voreingestellten Kennwert, der dem Identifikator entspricht, und dem Steuercode gemäß dem Abbildungsverhältnis zwischen den voreingestellten Kennwerten und den Steuercodes, und gemäß einer Verbindung zwischen den Identifikatoren; und Bestimmen des Steuercodes, der dem passenden voreingestellten Kennwert entspricht, wenn ein Kennwert zu dem voreingestellten Kennwert in dem Abbildungsverhältnis zwischen den voreingestellten Kennwerten und den Steuercodes passt.

10. Endgerätsteuersystem, aufweisend:

   ein Erhaltungsmodul (10), das so konfiguriert ist, dass es einen Kennwert eines elektromyographischen Signals erhält, wenn das elektromyographische Signal durch eine Erfassungsvorrichtung für elektromyographische Signale erfasst wird,
   wobei der Kennwert einen Durchschnittswert oder eine Varianz von Wavelet-Koeffizienten, die durch eine Durchführung einer Wavelet-Zerlegung des elektromyographischen Signals erhalten wird, darstellt; und
   ein Bestimmungsmodul (20), das so konfiguriert ist, dass es ein Abbildungsverhältnis zwischen voreingestellten Kennwerten und Steuercodes sucht, um zu bestimmen, ob es einen voreingestellten Kennwert gibt, der zu dem erhaltenen Kennwert passen kann, und Bestimmen eines Steuercodes, der dem passenden voreingestellten Kennwert entspricht, falls es einen passenden voreingestellten Kennwert gibt; und
   ein Antwortmodul (30), das so konfiguriert ist, dass es auf ein Steuersignal antwortet, das dem bestimmten Steuercode entspricht, wobei das Erhaltungsmodul (10) aufweist:

ein Abtastsubmodul (11), das so konfiguriert ist, dass es das elektromyographische Signal abtastet, so dass eine Mehrzahl an Abtastpunkten erhalten wird und ein Energiewert jedes Abtastpunkts erhalten wird;

ein Energiewerterhaltungssubmodul (12), das so konfiguriert ist, dass es einen Energiewert jedes Abtastpunkts erhält, wenn das elektromyographische Signal durch die Erfassungsvorrichtung für elektromyographische Signale erfasst wird;

ein Startpunkterhaltungssubmodul (13), das so konfiguriert ist, dass es einen Startpunkt eines ersten Segments des elektromyographischen Signals, das eine Mehrzahl an aufeinanderfolgenden Abtastpunkten als einen Startpunkt eines effektiven elektromyographischen Signals beinhaltet, wenn jeder der Energiewerte der aufeinanderfolgenden Abtastpunkte größer ist als eine erste Energieschwelle, und wenn die Anzahl der aufeinanderfolgenden Abtastpunkte größer ist als eine erste Anzahlschwelle;

ein Endpunkterhaltungssubmodul (14), das so konfiguriert ist, dass es einen Endpunkt eines zweiten Segments des elektromyographischen Signals, das eine Mehrzahl an aufeinanderfolgenden Abtastpunkten als einen Endpunkt des effektiven elektromyographischen Signals beinhaltet, wenn jeder der Energiewerte der aufeinanderfolgenden Abtastpunkte geringer ist als eine zweite Energieschwelle, und wenn die Anzahl der aufeinanderfolgenden Abtastpunkte, die in dem zweiten Segment enthalten sind, größer ist als eine zweite Anzahlschwelle; und

ein Extraktionssubmodul (15), das so konfiguriert ist, dass es den Kennwert des effektiven elektromyographischen Signals zwischen dem Startpunkt und dem Endpunkt extrahiert.

11. Endgerätsteuersystem nach Anspruch 10, wobei das Startpunkterhaltungssubmodul (13) ferner aufweist:

eine Erhaltungseinheit (131) für den ersten Energiewert, die so konfiguriert ist, dass sie den Energiewert des ersten Segments des elektromyographischen Signals, das die aufeinanderfolgenden Abtastungen enthält, erhält, wenn jeder der Energiewerte der aufeinanderfolgenden Abtastungen größer ist als die erste Energieschwelle, und wenn die Anzahl sukzessiver Abtastungen größer ist als die erste Anzahlschwelle; und

eine Startpunkterhaltungseinheit (132), die so konfiguriert ist, dass sie den Startpunkt des ersten Segments des elektromyographischen Signals als den Startpunkt des effektiven elektromyographischen Signals setzt, wenn der Energiewert des ersten Segments des elektromyographischen Signals größer ist als die erste Energieschwelle.

12. Endgerätsteuersystem nach Anspruch 10, wobei das Endpunkterhaltungssubmodul (14) ferner aufweist:

eine Erhaltungseinheit (141) für den zweiten Energiewert, die so konfiguriert ist, dass sie den Energiewert des zweiten Segments des elektromyographischen Signals, das die aufeinanderfolgenden Abtastpunkte enthält, erhält, wenn jeder der Energiewerte der aufeinanderfolgenden Abtastpunkte geringer ist als die zweite Energieschwelle, und wenn die Anzahl sukzessiver Abtastpunkte größer ist als die zweite Anzahlschwelle; und

eine Endpunkterhaltungseinheit (142), die so konfiguriert ist, dass sie den Endpunkt des zweiten Segments des elektromyographischen Signals als den Endpunkt des effektiven elektromyographischen Signals setzt, wenn der Energiewert des zweiten Segments des elektromyographischen Signals geringer ist als die zweite Energieschwelle.

13. Endgerätsteuersystem nach Anspruch 11, wobei das Endpunkterhaltungssubmodul (14) ferner aufweist:

eine Erhaltungseinheit (141) für den zweiten Energiewert, die so konfiguriert ist, dass sie den Energiewert des zweiten Segments des elektromyographischen Signals, das die aufeinanderfolgenden Abtastpunkte enthält, erhält, wenn jeder der Energiewerte der aufeinanderfolgenden Abtastpunkte geringer ist als die zweite Energieschwelle, und wenn die Anzahl sukzessiver Abtastpunkte größer ist als die zweite Anzahlschwelle; und

eine Endpunkterhaltungseinheit (142), die so konfiguriert ist, dass sie den Endpunkt des zweiten Segments des elektromyographischen Signals als den Endpunkt des effektiven elektromyographischen Signals setzt, wenn der Energiewert des zweiten Segments des elektromyographischen Signals geringer ist als die zweite Energieschwelle.

14. Endgerätsteuersystem nach Anspruch 10, ferner aufweisend:

ein Anzeigemodul (40), das so konfiguriert ist, dass es eine Auswahlschnittstelle, die eine Mehrzahl an Standardgestenbildern beinhaltet, anzeigt, wenn ein Gestenlernbefehl erfasst wird, so dass ein Benutzer ein Standardgestenbild auswählt und einen Auswahlbefehl auslöst, nachdem das Standardgestenbild ausgewählt wurde;

ein Sperrmodul (50), das so konfiguriert ist, dass es das Endgerät so sperrt, dass es das entsprechende Standardgestenbild gemäß dem Auswahlbefehl anzeigt, wenn der Auswahlbefehl empfangen wird;

ein Erhaltungsmodul (10), das so konfiguriert ist, dass es den Kennwert des elektromyographischen Signals erhält, wenn das elektromyographische Signal durch die Erfassungsvorrichtung für elektromyographische Signale erfasst wird; und

ein Aktualisierungsmodul (60), das so konfiguriert ist, dass es den Steuercode bestimmt, der dem Standardgestenbild entspricht, das gegenwärtig angezeigt wird, und den Kennwert aktualisiert, der dem bestimmten Steuercode zu dem erhaltenen Kennwert entspricht.

15. Endgerätesteuersystem nach Anspruch 10, ferner aufweisend:

ein Erfassungsmodul (70), das so konfiguriert ist, dass es das elektromyographische Signal von der Erfassungsvorrichtung für elektromyographische Signale erfasst, wenn der Gestenlernbefehl erfasst wird; und

ein Speichermodul (80);

wobei das Erhaltungsmodul (10) ferner so konfiguriert ist, dass es den Kennwert des elektromyographischen Signals erhält, und den Steuercode von dem Steuergerät empfängt, wenn das elektromyographische Signal durch die Erfassungsvorrichtung für elektromyographische Signale erfasst wird;

wobei das Speichermodul (80) so konfiguriert ist, dass es den erhaltenen Kennwert mit dem empfangenen Steuercode verbindet, wenn der Steuercode durch den Steuercode erfasst wird, und den erhaltenen Kennwert und den verbundenen Steuercode bei dem Abbildungsverhältnis zwischen den Kennwerten und den Steuercodes speichert.

16. Endgerätesteuersystem nach Anspruch 10, ferner aufweisend:

ein Anzeigemodul (40), das so konfiguriert ist, dass es die Standardgestenbilder aufeinanderfolgend anzeigt, wenn der Gestenlernbefehl erfasst wird;

ein Erfassungsmodul (70), das so konfiguriert ist, dass es das elektromyographische Signal erfasst, nachdem jedes Standardgestenbild angezeigt wurde;

ein Bestimmungsmodul (20), das so konfiguriert ist, dass es den Steuercode bestimmt, der dem Standardgestenbild entspricht, das gegenwärtig dargestellt wird, wenn das elektromyographische Signal erfasst wird; und

ein Aktualisierungsmodul (60), das so konfiguriert ist, dass es den Kennwert auf den erhaltenen Kennwert aktualisiert, der dem bestimmten Steuercode entspricht;

wobei das Anzeigemodul (40) ferner so konfiguriert ist, dass es ein nächstes Standardgestenbild anzeigt und weiter das Abbildungsverhältnis lernt.

17. Endgerätesteuersystem nach Anspruch 10, wobei das Bestimmungsmodul (20) ferner aufweist:

eine Erhaltungseinheit, die so konfiguriert ist, dass sie einen Identifikator der Erfassungsvorrichtung für elektromyographische Signale gemäß dem erfassten elektromyographischen Signal bestimmt, und gemäß dem Abbildungsverhältnis zwischen den voreingestellten Kennwerten und den Steuercodes und gemäß einer Verbindung zwischen den Identifikatoren ein Abbildungsverhältnis zwischen einem voreingestellten Kennwert, der dem Identifikator entspricht, und dem Steuercode erhält; und

eine Bestimmungseinheit, die so konfiguriert ist, dass sie den Steuercode bestimmt, der dem passenden voreingestellten Kennwert entspricht, wenn ein Kennwert zu dem voreingestellten Kennwert in dem Abbildungsverhältnis zwischen den voreingestellten Kennwerten und den Steuercodes passt.

**Revendications**

1. Procédé de commande de terminal comprenant :

un terminal obtenant (S10) une valeur caractéristique d'un signal électromyographique lorsque le signal électromyographique est détecté par un dispositif de détection de signal électromyographique, la valeur caractéristique étant une valeur moyenne ou une variance de coefficients d'ondelette obtenus en effectuant une décomposition en ondelettes du signal électromyographique ;

le terminal recherchant (S20) une relation de mappage entre des valeurs caractéristiques préétablies et des codes de commande pour déterminer s'il existe une valeur caractéristique préétablie qui peut correspondre à la valeur caractéristique obtenue, et déterminant un code de commande correspondant à la valeur caractéristique préétablie correspondante s'il existe une valeur caractéristique préétablie correspondante ; et

le terminal répondant (S30) à un signal de commande correspondant au code de commande déterminée, dans lequel l'étape dans laquelle « le terminal obtenant (S10) une valeur caractéristique d'un signal électromyographique lorsque le signal électromyographique est détecté par un dispositif de détection de signal électromyographique » comprend en outre de :

échantillonner (S11) le signal électromyographique pour obtenir une pluralité de points d'échantillonnage et obtenir une valeur d'énergie de chaque point d'échantillonnage lorsque le signal électromyographique est détecté par le dispositif de détection de signal électromyographique ;

établir (S12) un point de départ d'un premier segment du signal électromyographique comprenant une pluralité de points d'échantillonnage successifs en tant que point de départ d'un signal électromyographique effectif lorsque chacune des valeurs d'énergie des points d'échantillonnage successifs est supérieure à un premier seuil d'énergie, et lorsque le nombre de points d'échantillonnage successifs est supérieur à un premier seuil de nombre ;

établir (S13) un point final d'un second segment du signal électromyographique comprenant une pluralité de points d'échantillonnage successifs en tant que point final du signal électromyographique effectif lorsque chacune des valeurs d'énergie des points d'échantillonnage successifs est inférieure à un second seuil d'énergie, et lorsque le nombre des points d'échantillonnage successifs inclus dans le second segment est supérieur à un second seuil de nombre ; et

extraire (S14) la valeur caractéristique du signal électromyographique effectif entre le point de départ et le point final.

**2.** Procédé de commande de terminal selon la revendication 1, dans lequel l'étape consistant à « établir (S12) un point de départ d'un premier segment du signal électromyographique comprenant une pluralité de points d'échantillonnage successifs en tant que point de départ d'un signal électromyographique effectif lorsque chacune des valeurs d'énergie des points d'échantillonnage successifs est supérieure à un premier seuil d'énergie, et lorsque le nombre de points d'échantillonnage successifs est supérieur à un premier seuil de nombre », comprend en outre de :

obtenir (S121) la valeur d'énergie du premier segment du signal électromyographique comprenant les points d'échantillonnage successifs lorsque chacune des valeurs d'énergie des points d'échantillonnage successifs est supérieure au premier seuil d'énergie, et lorsque le nombre des points d'échantillonnage successifs est supérieur au premier seuil de nombre ; et

établir (S122) le point de départ du premier segment du signal électromyographique en tant que point de départ du signal électromyographique effectif lorsque la valeur d'énergie du premier segment du signal électromyographique est supérieure au premier seuil d'énergie.

**3.** Procédé de commande de terminal selon la revendication 1, dans lequel l'étape consistant à « établir (S13) un point final d'un second segment du signal électromyographique comprenant les points d'échantillonnage successifs en tant que point final du signal électromyographique effectif lorsque chacune des valeurs d'énergie des points d'échantillonnage successifs est inférieure à un second seuil d'énergie, et lorsque le nombre des points d'échantillonnage successifs est supérieur à un second seuil de nombre », comprend en outre de :

obtenir (S131) la valeur d'énergie du second segment du signal électromyographique comprenant les points d'échantillonnage successifs lorsque chacune des valeurs d'énergie des points d'échantillonnage successifs est inférieure au second seuil d'énergie, et lorsque le nombre des points d'échantillonnage successifs est supérieur au second seuil de nombre ; et

établir (S132) le point final du second segment du signal électromyographique en tant que point final du signal électromyographique effectif lorsque la valeur d'énergie du second segment du signal électromyographique est inférieure au second seuil d'énergie.

**4.** Procédé de commande de terminal selon la revendication 2, dans lequel l'étape consistant à « établir (S13) un point final d'un second segment du signal électromyographique comprenant les points d'échantillonnage successifs en tant que point final du signal électromyographique effectif lorsque chacune des valeurs d'énergie des points d'échantillonnage successifs est inférieure à un second seuil d'énergie, et lorsque le nombre des points d'échantillonnage successifs est supérieur à un second seuil de nombre » comprend en outre de :

obtenir (S131) la valeur d'énergie du second segment du signal électromyographique comprenant les points d'échantillonnage successifs lorsque chacune des valeurs d'énergie des points d'échantillonnage successifs est inférieure au second seuil d'énergie, et lorsque le nombre des points d'échantillonnage successifs est supérieur au second seuil de nombre ; et

établir (S132) le point final du second segment du signal électromyographique en tant que point final du signal électromyographique effectif lorsque la valeur d'énergie du second segment du signal électromyographique est inférieure au second seuil d'énergie.

5. Procédé de commande de terminal selon la revendication 1, comprenant en outre l'apprentissage de la relation de mappage entre les valeurs caractéristiques préétablies et les codes de commande comprenant de :

afficher (S40) une interface de sélection comprenant une pluralité d'images de gestuelle standard lorsqu'une instruction d'apprentissage de gestuelle est détectée, pour qu'un utilisateur sélectionne une image de gestuelle standard, et déclencher une instruction de sélection après la sélection de l'image de gestuelle standard ;

verrouiller (S50) le terminal en affichage de l'image de gestuelle standard correspondante en fonction de l'instruction de sélection lorsque l'instruction de sélection est reçue ;

obtenir (S60) la valeur caractéristique du signal électromyographique lorsque le signal électromyographique est détecté par le dispositif de détection de signal électromyographique ; et

déterminer (S70) le code de commande correspondant à l'image de gestuelle standard actuellement affichée, et mettre à jour la valeur caractéristique correspondant au code de commande déterminé en la valeur caractéristique obtenue.

6. Procédé de commande de terminal selon la revendication 1, comprenant en outre l'apprentissage de la relation de mappage entre les valeurs caractéristiques préétablies et les codes de commande, comprenant de :

détecter (S80) le signal électromyographique par le dispositif de détection de signal électromyographique lorsque l'instruction d'apprentissage de gestuelle est détectée ;

obtenir (S90) la valeur caractéristique du signal électromyographique, et recevoir le code de commande du terminal de commande lorsque le signal électromyographique est détecté par le dispositif de détection de signal électromyographique ; et

associer (S100) la valeur caractéristique obtenue au code de commande reçu lorsque le code de commande est détecté à partir du terminal de commande, et mémoriser la valeur caractéristique obtenue et le code de commande associé dans la relation de mappage entre les valeurs caractéristiques et les codes de commande.

7. Procédé de commande de terminal selon la revendication 6, dans lequel, lorsque l'instruction d'apprentissage de gestuelle est détectée, le procédé comprend en outre de :

déclencher l'instruction d'apprentissage de gestuelle lorsque le terminal détecte un même signal électromyographique un nombre de fois au cours d'un intervalle de temps préétabli et le nombre de fois est supérieur à un seuil préétabli.

8. Procédé de commande de terminal selon la revendication 1, comprenant en outre l'apprentissage de la relation de mappage entre les valeurs caractéristiques préétablies et les codes de commande comprenant de :

afficher successivement les images de gestuelle standard lorsque l'instruction d'apprentissage de gestuelle est détectée ;

détecter le signal électromyographique après l'affichage de chaque image de gestuelle standard ;

déterminer le code de commande correspondant à l'image de gestuelle standard actuellement affichée lorsque le signal électromyographique est détecté ;

mettre à jour la valeur caractéristique correspondant au code de commande déterminé en la valeur caractéristique obtenue ; et

afficher une image de gestuelle standard suivante et continuer l'apprentissage de la relation de mappage.

9. Procédé de commande de terminal selon la revendication 1, dans lequel l'étape dans laquelle « le terminal recherchant (S20) une relation de mappage entre des valeurs caractéristiques préétablies et des codes de commande pour déterminer s'il existe une valeur caractéristique préétablie qui peut correspondre à la valeur caractéristique obtenue, et déterminant un code de commande correspondant à la valeur caractéristique préétablie correspondante s'il existe une valeur caractéristique préétablie correspondante » comprend en outre de :

déterminer un identifiant du dispositif de détection de signal électromyographique en fonction du signal électromyographique détecté ;

obtenir une relation de mappage entre une valeur caractéristique préétablie correspondant à l'identifiant et le code de commande, en fonction de la relation de mappage entre les valeurs caractéristiques préétablies et les codes de commande, et en fonction d'une association entre les identifiants ; et

déterminer le code de commande correspondant à la valeur caractéristique préétablie correspondante, lorsqu'une valeur caractéristique correspond à la valeur caractéristique préétablie dans la relation de mappage entre les valeurs caractéristiques préétablies et les codes de commande.

10. Système de commande de terminal comprenant :

un module d'obtention (10) configuré pour obtenir une valeur caractéristique d'un signal électromyographique lorsque le signal électromyographique est détecté par un dispositif de détection de signal électromyographique, la valeur caractéristique étant une valeur moyenne ou une variance de coefficients d'ondelette obtenus en effectuant une décomposition en ondelettes du signal électromyographique ; et

un module de détermination (20) configuré pour rechercher une relation de mappage entre des valeurs caractéristiques préétablies et des codes de commande afin de déterminer s'il existe une valeur caractéristique préétablie qui peut correspondre à la valeur caractéristique obtenue, et déterminer un code de commande correspondant à la valeur caractéristique préétablie correspondante s'il existe une valeur caractéristique préétablie correspondante ; et

un module de réponse (30) configuré pour répondre à un signal de commande correspondant au code de commande déterminé, dans lequel le module d'obtention (10) comprend :

un sous-module d'échantillonnage (11) configuré pour échantillonner le signal électromyographique pour obtenir une pluralité de points d'échantillonnage et obtenir une valeur d'énergie de chaque point d'échantillonnage ;

un sous-module d'obtention de valeur d'énergie (12) configuré pour obtenir une valeur d'énergie de chaque point d'échantillon lorsque le signal électromyographique est détecté par le dispositif de détection de signal électromyographique ;

un sous-module d'obtention de point de départ (13) configuré pour établir un point de départ d'un premier segment du signal électromyographique comprenant une pluralité de points d'échantillonnage successifs en tant que point de départ d'un signal électromyographique effectif lorsque chacune des valeurs d'énergie des points d'échantillonnage successifs est supérieure à un premier seuil d'énergie, et lorsque le nombre des points d'échantillonnage successifs est supérieur à un premier seuil de nombre ;

un sous-module d'obtention de point final (14) configuré pour établir un point final d'un second segment du signal électromyographique comprenant une pluralité de points d'échantillonnage successifs en tant que point final du signal électromyographique effectif lorsque chacune des valeurs d'énergie des points d'échantillonnage successifs est inférieure à un second seuil d'énergie, et lorsque le nombre de points d'échantillonnage successifs inclus dans le second segment est supérieur à un second seuil de nombre ; et

un sous-module d'extraction (15) configuré pour extraire la valeur caractéristique du signal électromyographique effectif entre le point de départ et le point final.

11. Système de commande de terminal selon la revendication 10, dans lequel le sous-module d'obtention de point de départ (13) comprend en outre :

une première unité d'obtention de valeur d'énergie (131) configurée pour obtenir la valeur d'énergie du premier segment du signal électromyographique comprenant les échantillons successifs lorsque chacune des valeurs d'énergie des échantillons successifs est supérieure au premier seuil d'énergie, et lorsque le nombre des échantillons successifs est supérieur au premier seuil de nombre ; et

une unité d'obtention de point de départ (132) configurée pour établir le point de départ du premier segment du signal électromyographique en tant que point de départ du signal électromyographique effectif lorsque la valeur d'énergie du premier segment du signal électromyographique est supérieure au premier seuil d'énergie.

12. Système de commande de terminal selon la revendication 10, dans lequel le sous-module d'obtention de point final (14) comprend en outre :

une seconde unité d'obtention de valeur d'énergie (141) configurée pour obtenir la valeur d'énergie du second segment du signal électromyographique comprenant les points d'échantillonnage successifs lorsque chacune des valeurs d'énergie des points d'échantillonnage successifs est inférieure au second seuil d'énergie, et lorsque le nombre de points d'échantillonnage successifs est supérieur au second seuil de nombre ; et

une unité d'obtention de point final (142) configurée pour établir le point final du second segment du signal électromyographique en tant que point final du signal électromyographique effectif lorsque la valeur d'énergie du second segment du signal électromyographique est inférieure au second seuil d'énergie.

13. Système de commande de terminal selon la revendication 11, dans lequel le sous-module d'obtention de point final (14) comprend en outre :

une seconde unité d'obtention de valeur d'énergie (141) configurée pour obtenir la valeur d'énergie du second segment du signal électromyographique comprenant les points d'échantillonnage successifs lorsque chacune des valeurs d'énergie des points d'échantillonnage successifs est inférieure au second seuil d'énergie, et lorsque le nombre de points d'échantillonnage successifs est supérieur au second seuil de nombre ; et

une unité d'obtention de point final (142) configurée pour établir le point final du second segment du signal électromyographique en tant que point final du signal électromyographique effectif lorsque la valeur d'énergie du second segment du signal électromyographique est inférieure au second seuil d'énergie.

14. Système de commande de terminal selon la revendication 10, comprenant en outre :

un module d'affichage (40) configuré pour afficher une interface de sélection comprenant une pluralité d'images de gestuelle standard lorsqu'une instruction d'apprentissage de gestuelle est détectée, permettant à un utilisateur de sélectionner une image de gestuelle standard, et pour déclencher une instruction de sélection après la sélection de l'image de gestuelle standard ;

un module de verrouillage (50) configuré pour verrouiller le terminal en affichage de l'image de gestuelle standard correspondante conformément à l'instruction de sélection lorsque l'instruction de sélection est reçue ;

un module d'obtention (10) configuré pour obtenir la valeur caractéristique du signal électromyographique lorsque le signal électromyographique est détecté par le dispositif de détection de signal électromyographique ; et

un module de mise à jour (60) configuré pour déterminer le code de commande correspondant à l'image de gestuelle standard actuellement affichée, et pour mettre à jour la valeur caractéristique correspondant au code de commande déterminé en la valeur caractéristique obtenue.

15. Système de commande de terminal selon la revendication 10, comprenant en outre :

un module de détection (70) configuré pour détecter le signal électromyographique provenant du dispositif de détection de signal électromyographique lorsque l'instruction d'apprentissage de gestuelle est détectée ; et
un module de stockage (80) ;

dans lequel le module d'obtention (10) est en outre configuré pour obtenir la valeur caractéristique du signal électromyographique, et recevoir le code de commande provenant du terminal de commande lorsque le signal électromyographique est détecté par le dispositif de détection de signal électromyographique ;

le module de stockage (80) est configuré pour associer la valeur caractéristique obtenue au code de commande reçu lorsque le code de commande est détecté par le code de commande, et pour stocker la valeur caractéristique obtenue et le code de commande associé dans la relation de mappage entre les valeurs caractéristiques et les codes de commande.

16. Système de commande de terminal selon la revendication 10, comprenant en outre :

un module d'affichage (40) configuré pour afficher successivement les images de gestuelle standard lorsque l'instruction d'apprentissage de gestuelle est détectée ;

un module de détection (70) configuré pour détecter le signal électromyographique après l'affichage de chaque image de gestuelle standard ;

un module de détermination (20) configuré pour déterminer le code de commande correspondant à l'image de gestuelle standard actuellement affichée lorsque le signal électromyographique est détecté ; et

un module de mise à jour (60) configuré pour mettre à jour la valeur caractéristique correspondant au code de commande déterminé en la valeur caractéristique obtenue ;

dans lequel le module d'affichage (40) est en outre configuré pour afficher une image de gestuelle standard suivante et continuer l'apprentissage de la relation de mappage.

17. Système de commande de terminal selon la revendication 10, dans lequel le module de détermination (20) comprend en outre :

une unité d'obtention configurée pour déterminer un identifiant du dispositif de détection de signal électromyographique en fonction du signal électromyographique détecté, et pour obtenir une relation de mappage entre une valeur caractéristique préétablie correspondant à l'identifiant et le code de commande, en fonction de la relation de mappage entre les valeurs caractéristiques préétablies et les codes de commande, et en fonction d'une association entre les identifiants ; et

une unité de détermination configurée pour déterminer le code de commande correspondant à la valeur caractéristique préétablie correspondante, lorsqu'une valeur caractéristique correspond à la valeur caractéristique préétablie dans la relation de mappage entre les valeurs caractéristiques préétablies et les codes de commande.

DRAWINGS

Start

Obtain a characteristic value of an electromyographic signal when the electromyographic signal is detected by an electromyographic signal detecting device ⟋S10

Search a mapping relationship between preset characteristic values and control codes to determine whether there is a preset characteristic value which can match the obtained characteristic value, if yes, determine a control code corresponding to the matching preset characteristic value ⟋S20

Respond to a control signal corresponding to the determined control code ⟋S30

End

**FIG. 1**

Start

Sample the electromyographic signal to obtain a number of samples and obtain an energy value of each sample, when the electromyographic signal is detected ⟋ S11

Set a start point of a first segment of the electromyographic signal including a number of successive samples as a start point of an effective electromyographic signal, when each of the energy values of the successive samples is greater than a first energy threshold, and when the number of the successive samples is greater than a first number threshold ⟋ S12

Set an end point of a second segment of the electromyographic signal including the successive samples as an end point of the effective electromyographic signal, when each of the energy values of the successive samples is less than a second energy threshold, and when the number of the successive samples is greater than a second number threshold ⟋ S13

Extract the characteristic value of the effective electromyographic signal between the start point and the end point ⟋ S14

End

**FIG. 2**

```
                          ┌──────────┐
                          │  Start   │
                          └──────────┘
                                │
                                ▼
┌─────────────────────────────────────────────────────────────┐
│   Display a selecting interface including standard gesture    │      ─S40
│   pictures when a gesture learning command is detected for a  │
│   user to select a standard gesture picture, and trigger a    │
│   selecting command after the standard gesture picture is     │
│   selected                                                    │
└─────────────────────────────────────────────────────────────┘
                                │
                                ▼
┌─────────────────────────────────────────────────────────────┐
│   Lock the terminal into displaying the corresponding         │      ─S50
│   standard gesture picture according to the selecting command │
│   when the selecting command is received                      │
└─────────────────────────────────────────────────────────────┘
                                │
                                ▼
┌─────────────────────────────────────────────────────────────┐
│   Obtain the characteristic value of the electromyographic    │      ─S60
│   signal when the electromyographic signal is detected from   │
│   the electromyographic signal detecting signal               │
└─────────────────────────────────────────────────────────────┘
                                │
                                ▼
┌─────────────────────────────────────────────────────────────┐
│   Determine the control code corresponding to the standard    │      ─S70
│   gesture picture currently displayed, and update the         │
│   characteristic value corresponding to the determined        │
│   control code to the obtained characteristic value           │
└─────────────────────────────────────────────────────────────┘
                                │
                                ▼
                          ┌──────────┐
                          │   End    │
                          └──────────┘
```

**FIG. 3**

Start

Detect the electromyographic signal when the gesture learning command is detected — S80

Obtain the characteristic value of the electromyographic signal when the electromyographic signal is detected from the electromyographic signal detecting device, and receive the control code from the control terminal — S90

Associate the obtained characteristic value with the received control codewhen the control code is detected from the control code, and store the obtained characteristic value and the associated control code to the mapping relationship between the characteristic values and the control codes — S100

End

FIG. 4

Start

Obtains the energy value of the first segment of the electromyographic signal including the successive samples when each of the energy values of the successive samples is greater than the first energy threshold, and when the number of the successive samples is greater than the first number threshold — S121

Set the start point of the first segment of the electromyographic signal as the start point of the effective electromyographic signal when the energy value of the first segment of the electromyographic signal is greater than the first energy threshold — S122

End

**FIG. 5**

Start

Obtain the energy value of the second segment of the electromyographic signal including the successive samples when each of the energy values of a number of successive samples is less than the second energy threshold, and when the number of the successive samples is greater than the second number threshold ⟋ S131

Set the end point of the second segment of the electromyographic signal as the end point of the effective electromyographic signal when the energy value of the second segment of the electromyographic signal is less than the second energy threshold ⟋ S132

End

**FIG. 6**

Terminal control system

Obtaining module ⟋ 10

Determining modue ⟋ 20

Responding module ⟋ 30

**FIG. 7**

**FIG. 8**

**FIG. 9**

Terminal control system

Obtaining module — 10

Determining module — 20

Responding module — 30

Detecting module — 70

Storage module — 80

**FIG. 10**

— 13

Start point obtaining
sub-module

First energy value obtaining
unit — 131

Start point obtaining unit — 132

**FIG. 11**

14

End point obtaining
sub-module

Second energy value
obtaining unit

141

End point obtaining unit

142

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1408443 A1

- US 2009327171 A1

**Non-patent literature cited in the description**

- **M. B. I. REAZ.** *Techniques of EMG signal analysis: detection, processing, classification and applications*
- **ZHANG XU.** *An Adaptive Feature Extractor for Gesture SEMG Recognition*

- **BAOFENG SUN.** *The Pattern Recognition of Surface EMG Based on Wavelet Transform and BP Neural Network*